(19) Europäisches Patentamt
European Patent Office
Office européen des brevets

(11) EP 2 468 274 B1

(12) **EUROPÄISCHE PATENTSCHRIFT**

(45) Veröffentlichungstag und Bekanntmachung des Hinweises auf die Patenterteilung:
**01.07.2015 Patentblatt 2015/27**

(51) Int Cl.:
***A61K 31/27*** *(2006.01)* ***A61K 9/00*** *(2006.01)*
***A61K 9/70*** *(2006.01)*

(21) Anmeldenummer: **10194968.3**

(22) Anmeldetag: **14.12.2010**

(54) **Transdermales therapeutisches System zur Verabreichung eines Wirkstoffs**

Transdermal therapeutic system for application of an agent

Système thérapeutique transdermique pour l'enrichissement d'une substance active

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**27.06.2012 Patentblatt 2012/26**

(73) Patentinhaber: **Acino AG**
**83714 Miesbach (DE)**

(72) Erfinder:
- **Schröder, Britta**
**82008, Unterhaching (DE)**
- **Schurad, Björn**
**81667, München (DE)**
- **Hausner, Heike**
**83607, Holzkirchen (DE)**

(74) Vertreter: **Kalhammer, Georg et al**
**Lederer & Keller**
**Patentanwälte Partnerschaft mbB**
**Unsöldstrasse 2**
**80538 München (DE)**

(56) Entgegenhaltungen:
**EP-A1- 2 016 939** **WO-A1-94/04109**
**DE-A1- 19 918 106** **US-A1- 2008 175 890**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 468 274 B1

## Beschreibung

Gegenstand der vorliegenden Anmeldung ist ein System zur transdermalen Verabreichung eines Wirkstoffs, bevorzugt Rivastigmin, dessen physiologisch verträglichem Salz, Hydrat, Solvat oder Derivat, das für einen therapeutischen Anwendungszeitraum von mehreren Tagen geeignet ist.

Bei Rivastigmin handelt es sich um das Phenylcarbamat (S)-N-ethyl-3-[(1-dimethylamino)ethyl]-N-methyl-phenyl-carbamat der Formel I.

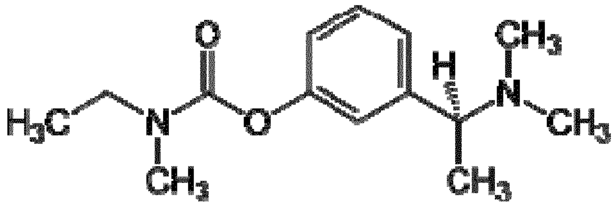

(I)

Es ist ein im zentralen Nervensystem wirkender Cholinesteraseinhibitor und ist daher ein Wirkstoff zur Behandlung von Alzheimer und Parkinsondemenz.

Rivastigmin kann als freie Base, aber auch als Säureadditionssalz, Hydrat, Solvat oder als sonstiges Derivat vorliegen. Diese Derivate sind, sofern nicht anders beschrieben, in der vorliegenden Erfindung mit der Bezeichnung "Rivastigmin" umfasst.

Eine bevorzugte Darreichungsform von Rivastigmin ist die perkutane Verabreichung mittels eines transdermalen therapeutischen Systems, also ein Transdermalpflaster. Ein Transdermalpflaster ist typischerweise eine kleine selbstklebende Bandage, welche den abzugebenden Wirkstoff enthält. Diese Bandagen können verschiedene Formen und Größen haben. Der einfachste Typ ist ein Klebe-Monolith, welcher einen Wirkstoffvorrat auf einem Träger (Deckschicht) umfasst. Dieser Wirkstoffvorrat wird typischerweise in Form einer Wirkstoffschicht aus dem Wirkstoff in einem pharmazeutisch akzeptablen, druckempfindlichen Klebstoff oder Haftkleber gebildet und steht mit der Hautoberfläche in Kontakt, wodurch der Wirkstoff durch transdermale Diffusion in den Körper des Patienten abgegeben wird.

Komplexere Pflaster sind Mehrfachlaminate oder Pflaster mit Wirkstoffvorrat, in welchen zwischen der Wirkstoffschicht und der Haut eine weitere Klebeschicht angeordnet sein kann.

Eine Darreichungsform über Transdermalpflaster von Rivastigmin wurde bereits im Grundpatent zu Rivastigmin, der GB 2203040 beschrieben. Das darin offenbarte Transdermalpflaster besteht aus einer Deckschicht und einer die Wirkstoffschicht bildenden Schicht. Neben dem Wirkstoff Rivastigmin sind in der Wirkstoffschicht ein hydrophiles Polymer, ein nicht-quellendes Acrylatpolymer und ein Weichmacher enthalten.

Nach Veröffentlichung der GB 2203040 wurden weitere transdermale therapeutische Systeme (TTS), die unter anderem Rivastigmin als Wirkstoff enthalten, entwickelt und beschrieben. In der WO 02/03969 ist ein TTS beschrieben, bei dem die wirkstoffenthaltende Matrixschicht zusätzlich hochdisperses Siliziumdioxid zur Erhöhung der Hautpermeation enthält.

In den Patentanmeldungen WO 2008/021113 und EP 2 016 939 werden Pflaster mit einem komplexeren Aufbau offenbart, welche eine Verabreichung von Rivastigmin über einen Zeitraum von bis zu sieben Tagen gewährleisten sollen. Diese Pflaster enthalten zwischen der den Wirkstoff enthaltenden Schicht und einer Klebeschicht zusätzlich eine die Abgabe des Wirkstoffs steuernde Membran.

In der DE 199 18 106 enthält die Wirkstoffschicht ein haftklebendes Polymer, welches Acrylsäure oder Methacrylsäureeinheiten mit einem definierten Gehalt an Carboxylgruppen aufweist, wodurch die Wasseraufnahmefähigkeit sowie die Toleranz saurer Polyacrylhaftkleber gegenüber Feuchtigkeit erhöht werden soll.

Die WO 2007/064407 A1 offenbart ein TTS mit einer auf Silikon basierenden Klebeschicht, wodurch eine Verbesserung hinsichtlich der Klebeeigenschaften, der Verträglichkeit und der Sicherheit bei der Rivastigmintherapie erreicht werden soll. Gemäß WO 2007/064407 A1 ist es besonders bevorzugt, dass die Reservoirschicht ein Antioxidationsmittel enthält (Seite 7, 4. Absatz). Entsprechend enthalten alle Formulierungen in den Beispielen das Antioxidationsmittel Vitamin E. Das dort verwendete Durotak® 387-2353 ist ein Polyacrylat, das Carboxylgruppen aufweist. Die Reservoirschicht soll nach WO 2007/064407 A1 auch als Penetrationsverbesserer verschiedene Substanzen wie z. B. Glycerin, Fettsäuren, etc. enthalten (Seite 7, 5. Absatz). Diese Substanzen enthalten meist freie Hydroxyl- oder Carboxylgruppen, die damit in der Polymermatrix der Reservoirschicht vorhanden sind. WO 2007/064407 A1 beschäftigt sich nicht besonders mit der Stabilität von Rivastigmin. Insbesondere lehrt WO 2007/064407 A1 nicht, bestimmte Polymere für die Polymermatrix der Reservoirschicht auszuwählen, um einem Abbau von Rivastigmin vorzubeugen.

US 2008/0044461 A1 offenbart TTS-Formulierungen mit Donepezil (siehe Beispiele). Rivastigmin wird auch erwähnt (Anspruch 7). US 2008/0044461 A1 offenbart keine Wirkstoffschicht mit einer Polymermatrix. Vielmehr wird die Freisetzung über eine Membran gesteuert (sog. Membranpflaster), nicht durch ein Polymergerüst, in das der Wirkstoff

eingebettet ist (sog. Matrixpflaster). Zudem ist es ein essentielles Merkmal von US 2008/0044461 A1, dass die Reservoirschicht einen Gelbildner und einen Permeationsverstärker enthält (siehe Anspruch 1). Als Permeationsverstärker werden Alkohole eingesetzt (siehe [0053]). Als Gelbildner werden Cellulosepolymere eingesetzt (siehe [0055]). Sowohl bei den Permeationsverstärkern als auch bei den Gelbildnern handelt es sich somit um Verbindungen mit freien Hydroxylgruppen, die im TTS in der Reservoirschicht vorhanden sind.

US 2007/0259028 A1 offenbart TTS-Formulierungen mit Donepezil (siehe Beispiele). Rivastigmin wird auch erwähnt (Anspruch 3). Es ist ein essentielles Merkmal von US 2007/0259028 A1, dass die Reservoirschicht einen mehrwertigen Alkohol enthält, z. B. Glycerin. Gemäß US 2007/0259028 A1 sind also zwangsläufig freie Hydroxylgruppen in der Polymermatrix der Reservoirschicht vorhanden.

US 2004/0086552 A1 offenbart TTS-Formulierungen mit einem Wirkstoff, der aus einer sehr langen Liste ausgewählt sein kann (siehe [0070] bis [0095]). Es werden sowohl Matrixpflaster als auch "Membranpflaster" offenbart (siehe [0057] bzw. [0058]). Für die Matrixpflaster lehrt US 2004/0086552 A1 nicht, bestimmte Polymere für die Matrix auszuwählen, um den Wirkstoff zu stabilisieren.

US 6,689,379 B1 offenbart TTS-Formulierungen mit einer besonderen Klebeschicht. Rivastigmin wird auch als möglicher Wirkstoff erwähnt. Die Wirkstoffschicht soll vorzugsweise eine Verbindung mit Hydroxylgruppen enthalten, siehe Anspruch 10. US 6,689,379 B1 lehrt nicht, bestimmte Polymere für die Polymermatrix der Reservoirschicht auszuwählen, um einem Abbau von Rivastigmin vorzubeugen.

US 2008/0175890 A1 beschreibt ein TTS zur Verabreichung von Sufentanil, das Polyisobutylen und Polybuten in der Klebeschicht enthält.

WO 94/04109 A1 beschreibt ein TTS zur Verabreichung von Nikotin, das Polyisobutylen und Polybuten in der Klebeschicht enthält.

Das Patent EP 1 047 409 berichtet jedoch von einem generellen Problem bei der Verabreichung von Rivastigmin durch ein TTS. Es wurde gefunden, dass der Wirkstoff gerade in Gegenwart von Sauerstoff zersetzungsanfällig ist. In der transdermalen Zusammensetzung welche in der GB 2203040 offenbart ist, zersetzt sich gemäß der Offenbarung in EP 1 047 409 das Rivastigmin auch trotz der Bildung einer geschlossenen Polymermatrix um den Wirkstoff und einer luftdichten Verpackung der Zusammensetzung. In der EP 1 047 409 wird das Problem der geringen Stabilität von Rivastigmin dadurch gelöst, dass der pharmazeutischen Zusammensetzung ein Antioxidationsmittel zugesetzt wird.

Bisher erlauben kommerziell erwerbliche TDS mit Rivastigmin lediglich eine kontinuierliche Verabreichung über 24 Stunden. Eine Aufgabe der Erfindung ist die Steuerung der Freigabe über das verlängerte Zeitintervall. Bei der transdermalen Verabreichung von Rivastigmin über einen längeren Zeitraum muss sichergestellt sein, dass (1) eine ausreichende chemische Stabilität des Wirkstoffs Rivastigmin gewährleistet ist, (2) eine ausreichende physikalische Stabilität (v.a. hinsichtlich Cold flow) des Systems gewährleistet ist und (3) das System eine hinreichende Klebkraft über die Applikationszeit aufweist.

Eine weitere Aufgabe der vorliegenden Erfindung ist es, therapeutische Zusammensetzungen enthaltend Rivastigmin für die transdermale Verabreichung zu finden, die für einen therapeutischen Anwendungszeitraum von mehreren Tagen geeignet ist.

## Zusammenfassung der Erfindung

Es wurde gefunden, dass Rivastigmin in Transdermalpflastern hinreichend stabil ist, wenn die Polymermatrix der Wirkstoffschicht keine freien Hydroxylgruppen oder Carboxylgruppen enthält. Die vorliegende Erfindung beruht unter anderem darauf, spezielle Polymere für die Polymermatrix auszuwählen, um dadurch einen Abbau von Rivastigmin zu verhindern bzw. zu minimieren.

Die vorliegende Erfindung stellt daher ein hinreichend stabiles TTS enthaltend Rivastigmin und ein Verfahren zur Herstellung desselben bereit, wie in den Ansprüchen definiert.

Ein erster Aspekt der vorliegenden Erfindung ist ein TTS zur Verabreichung von Rivastigmin für einen Anwendungszeitraum von mehreren Tagen, das folgende Komponenten umfasst:

a) eine Deckschicht,
b) eine auf der Deckschicht befindliche Wirkstoffschicht umfassend eine den Wirkstoff enthaltende Polymermatrix,
c) eine auf der Wirkstoffschicht befindliche, die Freisetzung des Rivastigmins kontrollierende Membran;
d) eine auf der Membran befindliche Klebeschicht umfassend einen Haftkleber, der ein Polyisobutylen oder ein Gemisch von mehreren Polyisobutylenen enthält; und
e) eine auf der Klebeschicht befindliche Abziehschicht,

wobei das Polymer/die Polymere der Wirkstoffschicht keine freien Hydroxylgruppen oder Carboxylgruppen enthält/enthalten, und wobei die Klebeschicht weiterhin ein Polybuten oder ein Gemisch von mehreren Polybutenen enthält.

Weiterhin wird die Verwendung von Polymeren oder Copolymeren, welche keine freien Hydroxylgruppen oder

Carboxylgruppen aufweisen, in einem TTS enthaltend Rivastigmin und ein TTS zur Behandlung von Alzheimer und Parkinsondemenz offenbart.

Während in der WO 2008/021113 die Klebstoffmaterialien Polyisobutylen, Polyacrylat und Klebstoffe auf Silikonbasis als gleichermaßen geeignet beschrieben werden, wurde vorliegend gefunden, dass sich Polyisobutylene als Haftkleber in der Klebeschicht besser eignen, und dass die Eigenschaften dieses Klebstoffs durch den Zusatz von Polybuten als Klebrigmacher weiter deutlich verbessert werden. Die vorliegende Erfindung stellt daher ein TTS enthaltend einen Wirkstoff, welches über verbesserte Klebeeigenschaften verfügt, und ein Verfahren zur Herstellung desselben bereit, wie in den Ansprüchen definiert.

Ein zweiter Aspekt der vorliegenden Offenbarung ist daher ein transdermales therapeutisches System zur Verabreichung eines Wirkstoffs durch die Haut, umfassend die zueinander in der folgenden Reihenfolge angeordneten Schichten:

a) eine Deckschicht,
b) eine Wirkstoffschicht umfassend eine den Wirkstoff enthaltende Polymermatrix,
c) eine Klebeschicht umfassend (1) einen Haftkleber, welcher aus einem Polyisobutylen oder einem Gemisch von mehreren Polyisobutylenen besteht und (2) einen Klebkraftverstärker, welcher aus Polybuten oder einem Gemisch von mehreren Polybutenen besteht, und
d) eine Abziehschicht.

Ein dritter Aspekt der vorliegenden Offenbarung ist ein TTS zur Verabreichung von Rivastigmin durch die Haut umfassend eine Deckschicht, eine Wirkstoffschicht, welche das Rivastigmin enthält, die gemäß dem zweiten Aspekt verbesserte Klebeschicht und eine Abziehschicht, für die Verabreichung von Rivastigmin über einen Zeitraum von mindestens zwei (z. B. zwei oder drei) Tagen, wobei sich zwischen der Wirkstoffschicht und der Klebeschicht eine die Freisetzung von Rivastigmin steuernde Membran befindet. Ein dritter Aspekt der vorliegenden Offenbarung ist daher ein transdermales therapeutisches System zur Verabreichung eines Wirkstoffs durch die Haut, umfassend die zueinander in der folgenden Reihenfolge angeordneten Schichten:

a) eine Deckschicht,
b) eine Wirkstoffschicht umfassend eine den Wirkstoff enthaltende Polymermatrix,
c) eine die Freisetzung des Rivastigmins kontrollierende Membran;
d) eine Klebeschicht umfassend (1) einen Haftkleber, welcher aus einem Polyisobutylen oder einem Gemisch von mehreren Polyisobutylenen besteht und (2) einen Klebkraftverstärker, welcher aus Polybuten oder einem Gemisch von mehreren Polybutenen besteht, und
e) eine Abziehschicht.

Ein vierter Aspekt der vorliegenden Erfindung ist ein TTS zur Verabreichung von Rivastigmin durch die Haut umfassend eine Deckschicht, eine Wirkstoffschicht, welche das Rivstigmin enthält, die gemäß dem zweiten Aspekt verbesserte Klebeschicht und eine Abziehschicht, wobei die Wirkstoffschicht keine freien Hydroxylgruppen oder Carboxylgruppen aufweist. Ein vierter Aspekt der vorliegenden Erfindung ist daher ein transdermales therapeutisches System zur Verabreichung eines Wirkstoffs durch die Haut, umfassend die zueinander in der folgenden Reihenfolge angeordneten Schichten:

a) eine Deckschicht,
b) eine Wirkstoffschicht umfassend eine den Wirkstoff enthaltende Polymermatrix,
c) eine Klebeschicht umfassend (1) einen Haftkleber, welcher aus einem Polyisobutylen oder einem Gemisch von mehreren Polyisobutylenen besteht und (2) einen Klebkraftverstärker, welcher aus Polybuten oder einem Gemisch von mehreren Polybutenen besteht, und
d) eine Abziehschicht,

wobei das Polymer/die Polymere der Wirkstoffschicht keine freien Hydroxylgruppen oder Carboxylgruppen enthält/enthalten.

Ein fünfter Aspekt der vorliegenden Offenbarung ist die Bereitstellung eines TTS zur Verabreichung von Rivastigmin durch die Haut umfassend eine Deckschicht, eine Wirkstoffschicht, welche das Rivastigmin enthält, die erfindungsgemäß verbesserte Klebeschicht und eine Abziehschicht, wobei zumindest die Wirkstoffschicht, bevorzugt jedoch das gesamte TTS, keine Antioxidationsmittel enthält. Ein fünfter Aspekt der vorliegenden Offenbarung ist daher ein transdermales therapeutisches System zur Verabreichung eines Wirkstoffs durch die Haut, umfassend die zueinander in der folgenden Reihenfolge angeordneten Schichten:

a) eine Deckschicht,

b) eine Wirkstoffschicht umfassend eine den Wirkstoff enthaltende Polymermatrix,

c) eine Klebeschicht umfassend (1) einen Haftkleber, welcher aus einem Polyisobutylen oder einem Gemisch von mehreren Polyisobutylenen besteht und (2) einen Klebkraftverstärker, welcher aus Polybuten oder einem Gemisch von mehreren Polybutenen besteht, und

d) eine Abziehschicht,

wobei zumindest die Wirkstoffschicht, bevorzugt jedoch das gesamte TTS, keine Antioxidationsmittel enthält.

Die oben genannten verschiedenen Aspekte können beliebig miteinander kombiniert werden. Der Wirkstoff im Sinne der vorliegenden Erfindung ist Rivastigmin oder ein physiologisch verträgliches Salz, Hydrat, Solvat oder Derivat davon.

## Ausführliche Beschreibung der Erfindung

### *Definitionen*

Ein "Antioxidationsmittel" im Sinne der vorliegenden Erfindung ist eine pharmazeutisch verträgliche Verbindung oder Zusammensetzung, die Oxidationsprozesse verlangsamt, hemmt, unterbricht und/oder aufhält. Antioxidationsmittel schließen insbesondere folgende Substanzen ein: Tocopherole und deren Ester, das Sesamol des Sesamöls, das Koniferylbenzoat des Benzoeharzes, die Nordihydroguajakharz- und -guajaretsäure (NDGA), die Gallate (Methyl-, Ethyl-, Propyl-, Amyl-, Butyl-, Lauryl- u.a. Gallate), das Butylhydroxdyanisol (BHT, auch Butyl-p-Kresol genannt); Ascorbinsäure und Salze und Ester davon, (z. B. Ascorbylpalmitat), Erythorbinsäure (iso-Ascorbinsäure) und Salze und Ester davon, Monothioglycerol, Natriumformaldehydsulfoxylat, Natriummetabisulfit, Natriumbisulfit, Natriumsulfit, Kaliummetabisulfit, butyliertes Hydroxyanisol, butyliertes Hydroxytoluol, Propionsäure.

Der Begriff "Tocopherol" schließt auch Tocopherol-Ester ein. Ein bekanntes Tocopherol ist $\alpha$-Tocopherol. Der Begriff "$\alpha$-Tocopherol" schließt Ester von $\alpha$-Tocopherol (z. B. $\alpha$-Tocopherol-Acetat) ein.

Eine "Polymermatrix" ist eine feste oder halbfeste Zusammensetzung mit dreidimensionaler Struktur, umfassend ein Polymer oder ein Polymergemisch. Die Polymermatrix wird auch als Polymergerüst bezeichnet, da die dreidimensionale Gerüst-Struktur in der Regel durch das Polymer oder Polymergemisch bereitgestellt wird. In die Polymermatrix können andere Stoffe, z. B. ein Wirkstoff, eingebettet sein. Vorzugsweise ist der Wirkstoff gleichmäßig in der Polymermatrix verteilt.

Im Folgenden werden die Merkmale der erfindungsgemäßen TTS detailliert beschrieben und, soweit es nicht ausdrücklich anders beschrieben ist, beziehen sich die jeweiligen Ausführungen zu den einzelnen Merkmalen auf sämtliche vorstehenden Aspekte der vorliegenden Erfindung.

Weiterhin können die vorstehenden Aspekte zu weiter bevorzugten Ausführungsformen beliebig kombiniert werden. So ist beispielsweise in einem bevorzugten TTS die Wirkstoffschicht frei von Tocopherolen, und das Polymer/die Polymere der Polymermatrix dieser Wirkstoffschicht enthält/enthalten weder Hydroxylgruppen noch Carboxylgruppen. Zudem befindet sich in dieser Ausführungsform zwischen der Wirkstoffschicht und der Klebeschicht bevorzugt eine die Freisetzung von Rivastigmin kontrollierende Membran, und das TTS eignet sich für einen Anwendungszeitraum von mindestens zwei, mindestens drei oder mindestens vier Tagen, z. B. für zwei bis vier Tage.

Der Wirkstoff Rivastigmin ist in dem erfindungsgemäßen TTS hinreichend stabil. "Hinreichend stabil" bedeutet, dass die Verunreinigungen des Wirkstoffs nach einem Monat Lagerung bei 40°C und 75 % relative Luftfeuchtigkeit insgesamt nicht mehr als 1 Gew.-% , bevorzugt nicht mehr als 0,5 Gew.-% betragen, bezogen auf den Sollgehalt an Wirkstoff in der Formulierung. Als Verunreinigungen des Wirkstoffs in der Formulierung gelten Abbauprodukte des Wirkstoffs Rivastigmin und mit dem Wirkstoff in die Formulierung eingetragene Verunreinigungen (z. B. Spuren von Zwischenprodukten aus der Herstellung des Wirkstoffs).

Die Stabilität bzw. die Menge an Verunreinigungen kann wie in Beispiel 4 beschrieben bestimmt werden. Vorzugsweise ist der Gesamtgehalt der Zersetzungsprodukte/Verunreinigungen nach drei Monaten Lagerung bei 40°C und 75 % relative Luftfeuchtigkeit weniger als 1 Gew.-%, bevorzugter weniger als 0,6 Gew.-%. Es ist auch bevorzugt, dass der Gesamtgehalt der Zersetzungsprodukte/Verunreinigungen nach sechs Monaten Lagerung bei 40°C und 75 % relative Luftfeuchtigkeit weniger als 1 Gew.-% beträgt. Es ist weiterhin bevorzugt, dass der Gesamtgehalt der Verunreinigungen nach einem Monat Lagerung bei 25°C und 60 % relative Luftfeuchtigkeit weniger als 0,25 Gew.-% beträgt. Es ist weiterhin bevorzugt, dass der Gesamtgehalt der Verunreinigungen nach drei und nach sechs Monaten Lagerung bei 25°C und 60 % relative Luftfeuchtigkeit weniger als 0,5 Gew.-% beträgt. Die Angaben von "Gew.-%" Verunreinigungen beziehen sich immer auf den Sollgehalt an Wirkstoff in der Formulierung, soweit nicht anders angegeben.

Die Anwendungsdauer eines erfindungsgemäßen TTS beträgt bevorzugt mindestens zwei oder mindestens drei Tage. In einer speziellen Ausführungsform ist das erfindungsgemäße TTS für eine Anwendungsdauer von 2 bis 4, 2 bis 5, 2 bis 6, 2 bis 7 oder 3 bis 8 Tagen geeignet.

Vorzugsweise enthält die Wirkstoffschicht, bevorzugter das gesamte TTS, kein Tocopherol. In einer weiteren Ausführungsform enthält die Wirkstoffschicht, vorzugsweise das gesamte TTS, kein Tocopherol und kein Butylhydroxdyanisol (BHT, auch Butyl-p-Kresol genannt). In einer weiteren Ausführungsform enthält die Wirkstoffschicht, vorzugsweise das gesamte TTS, kein Tocopherol, kein Butylhydroxdyanisol und kein Butylhydroxytoluol. In einer besonderen Ausführungsform enthält die Wirkstoffschicht, vorzugsweise das gesamte TTS, keines der folgenden Antioxidationsmittel: Tocopherole und dessen Ester, das Sesamol des Sesamöls, das Koniferylbenzoat des Benzoeharzes, die Nordihydroguajakharz- und -guajaretsäure (NDGA), die Gallate (Methyl-, Ethyl-, Propyl-, Amyl-, Butyl-, Lauryl- u.a. Gallate), das Butylhydroxdyanisol (BHT, auch Butyl-p-Kresol genannt); Ascorbinsäure und Salze davon, Ascorbylpalmitat, Erythorbinsäure (iso-Ascorbinsäure) und Salze davon, Monothioglycerol, Natriumformaldehydsulfoxylat, Natriummetabisulfit, Natriumbisulfit, Natriumsulfit, Kaliummetabisulfit, butyliertes Hydroxyanisol, butyliertes Hydroxytoluol, Propionsäure. In einer speziellen Ausführungsform enthält die Wirkstoffschicht, bevorzugter das gesamte TTS, überhaupt keine Antioxidationsmittel.

Es ist jedoch durchaus möglich, dass in dem TTS der vorliegenden Erfindung Antioxidationsmittel vorhanden sein können, sofern diese die Wirkungsweise des TTS nicht negativ beeinflussen. Es ist hierbei zu beachten, dass zur Stabilisierung von Rivastigmin gemäß einem Aspekt der vorliegenden Erfindung keine Antioxidationsmittel nötig sind. Jedoch könnten Antioxidationsmittel auch für andere Zwecke in den erfindungsgemäßen TTS zum Einsatz kommen. Es ist daher möglich, wenn auch nicht bevorzugt, dass das erfindungsgemäße TTS Antioxidationsmittel enthält, z. B. Tocopherole wie $\alpha$-Tocopherol und dessen Ester, Butylhydroxytoluol und Butylhydroxyanisol.

Die Gesamtmenge an Antioxidationsmittel im TTS der vorliegenden Erfindung ist üblicherweise weniger als 1 Gew.-% oder weniger als 0.1 Gew.-%, bevorzugter weniger als 0.05 Gew.-%, am bevorzugtesten weniger als 0.01 Gew.-%, jeweils bezogen auf das Gewicht der Gesamtformulierung (exklusive der Deck- und Abziehschicht).

*Aufbau des TTS*

Der Aufbau der erfindungsgemäßen TTS umfasst mehrere Schichten. Auf dem bei der Anwendung der Haut abgewandten Ende des TTS befindet sich die Deckschicht. An der der menschlichen Haut beim Gebrauch zugewandten Seite der Deckschicht befindet sich die Wirkstoffschicht. Weiterhin befindet sich an der der menschlichen Haut beim Gebrauch zugewandten Seite der Wirkstoffschicht die Klebeschicht. An der der menschlichen Haut beim Gebrauch zugewandten Seite der Klebeschicht befindet sich vor der Anwendung des TTS die Abziehschicht, welche unmittelbar vor dem Gebrauch des TTS entfernt wird. Erfindungsgemäß befindet sich zwischen der Wirkstoffschicht und der Klebeschicht eine die Freisetzung des Wirkstoffs kontrollierende Membran.

Die Fläche des erfindungsgemäßen TTS ist nicht besonders eingeschränkt. Üblicherweise beträgt die Fläche etwa 5 - 40 $cm^2$, kann jedoch durchaus größer oder kleiner sein.

Die Fläche der Deckschicht des erfindungsgemäßen TTS entspricht in einer Ausführungsform mindestens der Fläche der Wirkstoffschicht beziehungsweise der Klebeschicht. Sie kann jedoch auch größer als die der Wirkstoffschicht sein, sodass sie nicht nur die Wirkstoffschicht vollständig bedeckt, sondern auch über den Rand der Wirkstoffschicht hinausragt. In einer solchen Ausführungsform sollte jedoch entweder die Fläche der Klebeschicht gleich der Fläche der Deckschicht sein oder die der Haut zugewandten Seite der Deckschicht eine weitere Klebeschicht aufweisen, um zu gewährleisten, dass die gesamte der Haut zugewandten Oberfläche des TTS bei der Anwendung auf der Haut haftet. In einer anderen Ausführungsform ist die Deckschicht etwas kleiner als die Fläche der Wirkstoffschicht und/oder Klebeschicht.

*Wirkstoffschicht*

Die Wirkstoffschicht des erfindungsgemäßen TTS enthält Rivastigmin, eingebettet in eine Polymermatrix. Die Polymermatrix umfasst im wesentlichen keine Polymere oder Copolymere, die freie Hydroxylgruppen oder freie Carboxylgruppen enthalten. Vorzugsweise enthält die Polymermatrix im wesentlichen keine freien Hydroxylgruppen und keine freien Carboxylgruppen. Bevorzugter enthält die Polymermatrix im wesentlichen keine freien Aminogruppen, keine freien Hydroxylgruppen und keine freien Carboxylgruppen. Die Polymermatrix wird vorzugsweise aus Polymeren und/oder Copolymeren gebildet, die im wesentlichen keine freien Hydroxylgruppen und keine freien Carboxylgruppen enthalten. Noch bevorzugter wird die Polymermatrix aus Polymeren und/oder Copolymeren gebildet, die keine freien Aminogruppen, keine freien Hydroxylgruppen und keine freien Carboxylgruppen enthalten.

Geeignete Polymere oder Copolymere ohne freie funktionelle Gruppen, die die Polymermatrix bilden, sind bestimmte Polyacrylate, Acrylat-Vinylacetat Copolymere, Polyisobutylen und Styrol-Butadien Copolymere, welche einzeln oder als Mischung vorliegen können.

Als geeignete Polyacrylate, die im wesentlichen keine freien funktionellen Gruppen enthalten, können Polymere (Homopolymere, Copolymere und Block-Copolymere) auf Basis von Acrylsäureestern und/oder Methacrylsäureestern verwendet werden. Als Monomere zur Herstellung geeigneter Polyacrylate kommen dabei insbesondere n-Butylacrylat,

n-Butylmethacrylat, Ethylacrylat, 2-Ethylhexylacrylat, Ethylmethacrylat, Methylacrylat, Methylmethacrylat, tert-Butylacrylat, sec-Butylacrylat, tert-Butylmethacrylat, Cyclohexylmethacrylat, 2-Ethylhexylmethacrylat, Isobornylmethacrylat, Isobutylmethacrylat, Isopropylacrylat, Isopropylmethacrylat und Mischungen dieser Monomere in Frage. Es handelt sich bei diesen Monomeren um Ester der Acryl- bzw. Methacrylsäure, die lineare, verzweigte oder cyclische aliphatische $C_1$-$C_{12}$- Substituenten ohne sonstige freie funktionelle Gruppen tragen. Auch Vinylacetat kann als Co-Monomer zusammen mit mindestens einem dieser Monomere zur Herstellung des Polyacrylats verwendet werden.

Die Polymermatrix besteht vorzugsweise aus einem oder mehreren Polyacrylaten, die im wesentlichen keine freien funktionellen Gruppen enthalten. Bevorzugter besteht die Polymermatrix aus Polyacrylaten, die durch Polymerisierung von Acrylsäureestern und/oder Methacrylsäureestern hergestellt wurden. In einer besonderen Ausführungsform besteht die Polymermatrix aus Polyacrylaten, die durch Polymerisierung von Acrylsäureestern und/oder Methacrylsäureestern hergestellt wurden, wobei die Acrylsäureester und/oder Methacrylsäureester ausgewählt sind aus der Gruppe bestehend aus n-Butylacrylat, n-Butylmethacrylat, Ethylacrylat, 2-Ethylhexylacrylat, Ethylmethacrylat, Methylacrylat, Methylmethacrylat, tert-Butylacrylat, sec-Butylacrylat, tert-Butylmethacrylat, Cyclohexylmethacrylat, 2-Ethylhexylmethacrylat, Isobornylmethacrylat, Isobutylmethacrylat, Isopropylacrylat, Isopropylmethacrylat und Mischungen davon. In einer anderen Ausführungsform besteht die Polymermatrix im wesentlichen aus Polyacrylaten, die durch Copolymerisierung von Acrylsäureestern und/oder Methacrylsäureestern mit Vinylacetat hergestellt wurden, wobei die Acrylsäureester und/oder Methacrylsäureester ausgewählt sind aus der Gruppe bestehend aus n-Butylacrylat, n-Butylmethacrylat, Ethylacrylat, 2-Ethylhexylacrylat, Ethylmethacrylat, Methylacrylat, Methylmethacrylat, tert-Butylacrylat, sec-Butylacrylat, tert-Butylmethacrylat, Cyclohexylmethacrylat, 2-Ethylhexylmethacrylat, Isobornylmethacrylat, Isobutylmethacrylat, Isopropylacrylat, Isopropylmethacrylat und Mischungen davon. Besonders bevorzugt sind Copolymere, die aus den Startmonomeren 2-Ethylhexylacrylat und Vinylacetat hergestellt werden, z. B. ein Acrylat-Vinylacetat Copolymer, welches zu jeweils 50 % aus den Startmonomeren 2-Ethylhexylacrylat und Vinylacetat hergestellt wird (Duro-Tak® 87-4098). Ebenfalls bevorzugt ist das Acrylat-Polymer Duro-Tak® 87-9088 (ebenfalls ein Acrylat-Polymer ohne freie funktionelle Gruppen), erhältlich von der Firma Henkel. In einer speziellen Ausführungsform wird für die Polymermatrix das Acrylat-Polymer Duro-Tak® 87-900A oder Duro-Tak® 87-9301 eingesetzt.

Der Gesamtanteil von Monomeren, die freie Hydroxylgruppen oder freie Carboxylgruppen enthalten (z. B. Acrylsäure, Methacrylsäure und Ester der Acrylsäure bzw. Methacrylsäure, welche funktionelle Gruppen tragen, insbesondere die hydroxylgruppenhaltigen Ester), liegt unterhalb von 1 Gew. -%, vorzugsweise unterhalb von 0,5 Gew. -%, bevorzugter unterhalb von 0,2 ,Gew.-%; bezogen auf das Monomerengemisch, aus der die Polymermatrix hergestellt wird. In einer besonderen Ausführungsform liegt der Gesamtanteil dieser Monomere unterhalb von 0,1 Gew. -%. In einer besonderen Ausführungsform sind keine freien Hydroxylgruppen und keine freien Carboxylgruppen im Monomerengemisch enthalten.

Ein TTS, welches als wirkstoffhaltige Polymermatrix Polyacrylate enthält, die im wesentlichen frei von Hydroxylgruppen und Carboxylgruppen sind, wurde zwar in der WO 03/017988 A1 bereits beschrieben, jedoch nicht in Verbindung mit dem Wirkstoff Rivastigmin. Die in der WO 03/017988 beschriebene Aufgabe war es, den Nachteil der geringen Wirkstoffausnutzung eines TTS zu lösen. Gemäß dieser Offenbarung wurde diese Aufgabe durch Polymermatrices gelöst, welche im Idealfall frei von Hydroxylgruppen oder Carboxylgruppen sind. Der Wirkstoff Rivastigmin wird in dieser Druckschrift nicht erwähnt, geschweige denn eine die Stabilität von Rivastigmin steigernde Wirkung beschrieben.

Die Wirkstoffschicht enthält gemäß einem Aspekt der Erfindung im wesentlichen keine Polymeren oder Copolymeren, die freie Hydroxylgruppen oder freie Carboxylgruppen enthalten. Vorzugsweise enthält die Wirkstoffschicht im wesentlichen keine freien Hydroxylgruppen und keine freien Carboxylgruppen. Bevorzugter enthält die Wirkstoffschicht im wesentlichen keine freien Aminogruppen, keine freien Hydroxylgruppen und keine freien Carboxylgruppen. In einer besonderen Ausführungsform enthält auch die Klebeschicht im wesentlichen keine Polymeren oder Copolymeren, die freie Hydroxylgruppen oder freie Carboxylgruppen enthalten. Vorzugsweise enthält die Klebeschicht im wesentlichen keine freien Hydroxylgruppen und keine freien Carboxylgruppen. Bevorzugter enthält die Klebeschicht im wesentlichen keine freien Aminogruppen, keine freien Hydroxylgruppen und keine freien Carboxylgruppen.

Die Wirkstoffschicht enthält vorzugsweise 30 - 50 Gew.-% Rivastigmin und 50 - 70 Gew.-% Polymermatrix, bezogen auf das Gesamtgewicht der Wirkstoffschicht. In einer besonders bevorzugten Ausführungsform des erfindungsgemäßen TTS enthält die Wirkstoffschicht etwa 40 Gew.-% Rivastigmin und etwa 60 Gew.-% Polymermatrix. Bevorzugt enthält die Wirkstoffschicht neben dem Wirkstoff und der Polymermatrix keine weiteren Bestandteile. Es ist jedoch möglich, dass zusätzlich weitere im Stand der Technik bekannte Zusatzstoffe in der Wirkstoffschicht enthalten sind. So können beispielsweise Weichmacher oder Gelbildner zusätzlich in der Wirkstoffschicht vorhanden sein.

Die absolute Menge an Rivastigmin hängt von verschiedenen Faktoren, insbesondere von der Größe des einzusetzenden TTS, dem Flächengewicht und der Wirkstoffkonzentration in der Wirkstoffschicht ab. Die Flächengewichte der getrockneten Wirkstoffschicht-Matrix liegen bevorzugt in einem Bereich von 20 - 100 $g/m^2$, weiter bevorzugt in einem Bereich von 25 - 80 $g/m^2$ und noch weiter bevorzugt in einem Bereich von 30 - 70 $g/m^2$. Die Wirkstoffschicht kann eine Dicke (Trockendicke) im Bereich von 20 - 400 $\mu$m, oder 30 - 200 $\mu$m oder 40 - 100 $\mu$m aufweisen. Auch

andere Dicken als die vorstehend genannten sind möglich.

*Zwischenmembran*

Die gemäß der vorliegenden Erfindung zwischen Wirkstoffschicht und Klebeschicht liegende, die Freisetzung des Wirkstoffs kontrollierende Membran (auch als "Kontrollmembran" bezeichnet) besteht bevorzugt aus einem Polyolefin, wie beispielsweise Polypropylen (z.B. Celgard® 2400) oder besonders bevorzugt aus Polyethylen (z.B. CoTran™ 9719 oder CoTran™ 9720) oder weiter bevorzugt aus Polyethylen mit einem Vinylacetat-Anteil von 4,5 bis 19 % (z.B. CoTran™ 9707, CoTran™ 9702, CoTran™ 9728). Desweiteren können die Membranen eine Porosität von bis zu 90 % aufweisen (z.B. Solupor® 10P05A, Celgard® 2400).

Es können poröse Membranen oder kohärente Membranen eingesetzt werden.

Die Porosität der porösen Membranen kann bis zu etwa 90% betragen. Daten bevorzugter poröser Membranen sind:

Solupor® 10P05A (Polyethylen, Porosität: 83%, Dicke: 60 μm)
Celgard® 2400 (Polypropylen, Porosität: 41%, Dicke: 25 μm)

Daten bevorzugter kohärenter Membranen sind:

CoTran™ 9719 (Polyethylen, Dicke: 43,2 μm)
CoTran™ 9720 (Polyethylen, Dicke: 76,2 μm)
CoTran™ 9707 (Polyethylen mit 4,5% Vinylacetat, Dicke: 50,8 μm)
CoTran™ 9702 (Polyethylen mit 9,0% Vinylacetat, Dicke: 50,8 μm)
CoTran™ 9728 (Polyethylen mit 19,0% Vinylacetat, Dicke: 50,8 μm)

Die Membran ist üblicherweise zwischen 0,01 und 0,15 mm dick. Die bevorzugte Dicke der Membran beträgt 0,025 bis 0,080 mm.

Bevorzugt wird erfindungsgemäß eine kohärente Membran, bestehend im wesentlichen aus Polyethylen, mit einer Dicke von etwa 40 bis 80 μm (z. B. CoTran™ 9720) eingesetzt. Ebenfalls bevorzugt wird erfindungsgemäß eine kohärente Membran, bestehend im wesentlichen aus Polyethylen mit etwa 19,0% Vinylacetat (z. B. CoTran™ 9728) eingesetzt. Am bevorzugtesten wird erfindungsgemäß eine kohärente Membran, bestehend im wesentlichen aus Polyethylen, mit einer Dicke von etwa 40 bis 50 μm (z. B. CoTran™ 9719) eingesetzt.

Die Membran ist der essentielle Formulierungsbestandteil zur Gewährleistung einer kontrollierten mehrtägigen Abgabe des Wirkstoffs. Die Arzneistoffabgabe kann unter anderem durch den Vinylacetat-Anteil in der Membran gesteuert werden.

*Klebeschicht*

Die Klebeschicht des erfindungsgemäßen TTS enthält Polyisobutylen als Haftkleber und Polybuten um die Klebkraft zu verbessern. Polyisobutylen ist ein selbstklebender Haftkleber, der nicht aushärtet und daher seine klebenden Eigenschaften über einen langen Zeitraum beibehält. Bevorzugterweise werden Polyisobutylene mit unterschiedlichem mittlerem Molekulargewicht als Mischung eingesetzt. Polyisobutylen ist in verschiedenen mittleren Molekulargewichten erhältlich. Der Ausdruck "mittleres Molekulargewicht" im Zusammenhang mit Polyisobutylen bezieht sich in der vorliegenden Anmeldung auf das sog. Viskositätsmittel $M_v$. Das Viskositätsmittel $M_v$ wird aus der Lösungsviskosität einer Lösung des Polyisobutylens in Isooctan bei 20 °C bestimmt. Als Meßgerät dient ein Ubbelohde-Viskosimeter. Das Viskositätsmittel $M_v$ errechnet sich aus folgender Formel:

$$M_v = 0.65 \sqrt{\frac{J_o \times 10^2}{3.06}}$$

Die Ermittlung des zur Bestimmung des Viskositätsmittels $M_v$ benötigten Staudingerindex $J_o$ erfolgt nach der Schulz-Blaschke-Beziehung aus der gemessenen spezifischen Viskosität $\eta_{SP}$ und der Lösungskonzentration.

$$J_o = \eta_{sp}/c\ (1 + 0.31 \times \eta_{sp})\ cm^3/g$$ (Schulze-Blaschke-Beziehung)

Die spezifische Viskosität $\eta_{SP} = t/t_0 - 1$, wobei t und $t_0$ die Flusszeit der Lösung bzw. des Lösungsmittels (jeweils mit Hagenbach-Couette-Korrektur) sind, und c die Konzentration der Lösung in g/$cm^3$ ist. Gegebenenfalls kann die Vorschrift DIN 53728 ergänzend herangezogen werden.

Geeignete mittlere Molekulargewichte $M_v$ von Polyisobutylen liegen beispielsweise im Bereich von etwa 40.000 g/mol bis etwa 4.000.000 g/mol. Eine mögliche Mischung ist die von (1) Polyisobutylen mit einem mittleren Molekulargewicht $M_v$ von etwa 40.000 g/mol (z. B. Oppanol® B10, erhältlich von der Firma BASF) und (2) Polyisobutylen mit einem mittleren Molekulargewicht $M_v$ von über etwa 1.000.000 g/mol (z. B. Oppanol® B100, erhältlich von der Firma BASF, mit einem mittleren Molekulargewicht $M_v$ von etwa 1.110.000 g/mol). Es liegt innerhalb der Kenntnis eines Fachmanns, die verschiedenen Molekulargewichte im geeigneten Verhältnis zu mischen, um die gewünschten Eigenschaften der Klebeschicht zu erzielen.

Das Polyisobutylen in der Klebeschicht kann eine Molekulargewichtsverteilung aufweisen, die ein erstes relatives Maximum zwischen 30.000 g/mol und 100.000 g/mol, und ein zweites relatives Maximum zwischen 300.000 g/mol und 500.000 g/mol aufweist. Bevorzugter liegt das erste relative Maximum zwischen 35.000 g/mol und 50.000 g/mol und, unabhängig davon, dass zweite relative Maximum zwischen 350.000 g/mol und 450.000 g/mol. Am bevorzugtesten liegt das erste relative Maximum bei etwa 40.000 g/mol, und, unabhängig davon, das zweite relative Maximum bei etwa 400.000 g/mol.

Das Polyisobutylengemisch des Haftklebers kann dadurch erhalten werden, dass man ein erstes Polyisobutylenpolymer mit einem mittleren Molekulargewicht $M_v$ zwischen 30.000 g/mol und 100.000 g/mol mit einem zweiten Polyisobutylenpolymer mit einem mittleren Molekulargewicht $M_v$ zwischen 300.000 g/mol und 500.000 g/mol mischt. Das erste Polyisobutylenpolymer hat vorzugsweise ein mittleres Molekulargewicht $M_v$ zwischen 35.000 g/mol und 50.000 g/mol, am bevorzugtesten von etwa 40.000 g/mol. Das zweite Polyisobutylenpolymer hat vorzugsweise ein mittleres Molekulargewicht $M_v$ zwischen 350.000 g/mol und 450.000 g/mol, am bevorzugtesten von etwa 400.000 g/mol.

Die bevorzugteste Mischung ist die von (1) Polyisobutylen mit einem mittleren Molekulargewicht $M_v$ von etwa 40.000 g/mol (zum Beispiel Oppanol® B10 SFN, erhältlich von der Firma BASF) und (2) Polyisobutylen mit einem mittleren Molekulargewicht $M_v$ von etwa 400.000 g/mol (zum Beispiel Oppanol® B50 SF, erhältlich von der Firma BASF).

Die Anteile der beiden Polyisobutylenpolymere in dem Gemisch können variieren. Das Gewichtsverhältnis des ersten Polyisobutylenpolymer zum zweiten Polyisobutylenpolymer in dem Gemisch kann 10:1 bis 1:10 betragen, vorzugsweise 2:1 bis 1:2, am bevorzugtesten 3:2 bis 2:3. In besonders bevorzugten Ausführungsformen besteht das Polyisobutylenpolymer des Haftklebers aus 40 bis 60 Gew.-% Oppanol® B10 (zum Beispiel Oppanol® B10 SFN) und 60 bis 40 Gew.-% Oppanol® B50SF.

Erfindungsgemäß enthält die verbesserte Klebeschicht zusätzlich Polybuten. Geeignete mittlere Molekulargewichte $M_N$ von Polybuten liegen beispielsweise im Bereich von etwa 500 bis 10.000 g/mol. Auch Polybuten liegt bevorzugt in einer Mischung von unterschiedlichen durchschnittlichen Molekulargewichten vor. Eine bevorzugte Mischung ist die von (1) Polybuten mit einem mittleren Molekulargewicht $M_N$ von etwa 900 g/mol (z.B. Indopol® H-100, mit einem mittleren Molekulargewicht $M_N$ = 910 g/mol, erhältlich von der Firma Ineos) und (2) Polybuten mit einem mittleren Molekulargewicht $M_N$ von etwa 6.000 g/mol (z.B. Indopol® H-18000, erhältlich von der Firma Ineos).

Eine andere bevorzugte Mischung ist die von (1) Polybuten mit einem mittleren Molekulargewicht $M_N$ von etwa 900 g/mol (z.B. Indopol® H-100, mit einem mittleren Molekulargewicht $M_N$ = 910 g/mol, erhältlich von der Firma Ineos) und (2) Polybuten mit einem mittleren Molekulargewicht $M_N$ von etwa 2.500 g/mol (z.B. Indopol® H-1900, erhältlich von der Firma Ineos).

Besonders bevorzugt ist die Mischung von (1) Polybuten mit einem mittleren Molekulargewicht $M_N$ von etwa 2.500 (z.B. Indopol® H-1900, erhältlich von der Firma Ineos) und (2) Polybuten mit einem mittleren Molekulargewicht $M_N$ von etwa 6.000 g/mol (z.B. Indopol® H-18000). Es liegt innerhalb der Kenntnis eines Fachmanns, die verschiedenen Molekulargewichte im geeigneten Verhältnis zu mischen, um im System die gewünschten Eigenschaften der Klebeschicht hinsichtlich Klebkraft und Freisetzungsrate des Wirkstoffs zu erzielen.

Die Anteile zweier Polybutene in der Klebeschicht können variieren. Das Gewichtsverhältnis des ersten Polybutens zum zweiten Polybuten beträgt bevorzugt 2:1 bis 1:2, am bevorzugtesten 3:2 bis 2:3. In einer besonders bevorzugten Ausführungsform besteht das Polybutenpolymer in der Klebeschicht aus ewas 40 Gew.-% Polybuten mit einem mittleren Molekulargewicht $M_N$ von etwa 2.500 g/mol und aus etwa 60 Gew.-% Polybuten mit einem mittleren Molekulargewicht $M_N$ von 6.000 g/mol.

Das "mittlere Molekulargewicht $M_N$" ist das Zahlenmittel der Molmasse und kann bestimmt werden gemäß "American Standard" ASTM D3536-91 oder ASTM D5296-05.

Die erfindungsgemäßen Bestandteile der verbesserten Klebeschicht Polyisobutylen und Polybuten liegen bevorzugt im Gewichtsverhältnis von 4:1 bis 1:2, bevorzugter 3:1 bis 1:2 vor. Beispielsweise können Polyisobutylen und Polybuten im Gewichtsverhältnis von etwa 1:1 vorliegen. In einer anderen Ausführungsform liegen Polyisobutylen und Polybuten im Gewichtsverhältnis von etwa 7:3 vor.

Die Klebeschicht enthält bevorzugt etwa 10 bis etwa 90 Gew.-%, vorzugsweise etwa 25 bis etwa 75 Gew.-%, am bevorzugtesten etwa 40 bis etwa 70 Gew.-% Polyisobutylen (z. B. ewa 50 Gew.-% oder etwa 70 Gew.-%), bezogen

auf das Gesamtgewicht der Klebeschicht. Diese Anteile bezeichnen den Gesamtgehalt an Polyisobutylen bzw. Polyisobutylen-Gemisch in der Klebeschicht. Die Klebeschicht enthält ebenfalls bevorzugt etwa 5 bis ewa 80 Gew.-%, vorzugsweise etwa 15 bis etwa 60 Gew.-%, am bevorzugtesten etwa 25 bis etwa 50 Gew.-% Polybuten (z. B. etwa 50 Gew.-% oder etwa 30 Gew.-%), bezogen auf das Gesamtgewicht der Klebeschicht. Diese Anteile bezeichnen den Gesamtgehalt an Polybuten bzw. Polybuten -Gemisch in der Klebeschicht.

In einer bevorzugten Ausführungsform enthält die Klebeschicht kein Acrylatpolymer und kein Acrylat-Copolymer.

Die Dicke der Klebeschicht (Trockendicke) ist nicht besonders eingeschränkt. Sie kann in einem Bereich von etwa 10 - 300 μm, oder in einem Bereich von 70 -140 μm liegen. Die absolute Menge der Klebeschicht kann etwa 10-50 $g/m^2$, oder 20 - 40 $g/m^2$ betragen, ohne darauf eingeschränkt zu sein.

Die Klebeschicht enthält im allgemeinen 60 - 100 Gew.-% des Haftklebers (z. B. Polyisobutylen) bzw. des Haftklebergemisches einschließlich Klebkraftverstärker (z.B. Polybuten). Weitere mögliche Bestandteile der Klebeschicht sind Weichmacher und Gelbildner.

Geeignete Weichmacher sind im Stand der Technik bekannt, wobei es sich bevorzugt um Mineralöl, Neutralöl, Paraffin, Leinsamenöl, Octylpalmitat, Squalen, Squalan, Silikonöl, Isobutylmyristat, Isostearylalkohol und/oder Oleylalkohol, besonders bevorzugt um Mineralöl, Neutralöl und/oder Paraffin handelt. Mineralöle sind farblose, klare Kohlenwasserstoffe. Sie werden aus den oberhalb von etwa 300°C siedenden Destillationsfraktionen des Erdöls gewonnen und durch Abkühlen von festen Kohlenwasserstoffen befreit. Durch geeignete Fraktionierung können Mineralöle gewonnen werden, die etwa bei Körpertemperatur, also bei etwa 35 - 37°C flüssig sind und bei tiefen Temperaturen, insbesondere bei Temperaturen unter 20°C fest sind. Die Auswahl des Mineralöls mit einem Verflüssigungspunkt von etwa 30 - 35°C ist bevorzugt. Besonders bevorzugt sind Paraffine und Mineralöle, welche den Anforderungen der Ph. Eur. 6 und/oder der USP 32-NF 27 entsprechen.

Der Weichmacher ist in der Klebeschicht im allgemeinen in einer Menge im Bereich von 0 - 40 Gew.-%, oder von 1-10 Gew.-%, oder im Bereich von 2-5 Gew.-%, beispielsweise 2 Gew.-% bezogen auf das Gesamtgewicht der Klebeschicht, vorhanden.

Bei dem Gelbildner handelt es sich bevorzugt um einen Gelbildner mit partikulärer Struktur, welcher auf seiner Oberfläche eine hohe Konzentration polarer Gruppen aufweist. Diese verursachen zu den Ölen hin entsprechend hohe Grenzflächenspannungen, die durch Agglomeration der Partikel untereinander zu Gelgerüsten teilweise kompensiert werden. Demzufolge sind die Gelgerüste immer umso fester, je größer der Polaritätsunterschied zwischen den Ölen und der Gerüstbildneroberfläche ist. Bevorzugt wird erfindungsgemäß als Gelbildner hochdisperses Siliziumdioxid oder pyrogene Kieselsäure eingesetzt. Die Größe der Partikel bewegt sich bevorzugt im Nanometerbereich und liegt zum Beispiel im Bereich von 400 - 1500 nm, insbesondere im Bereich von 500 - 1000 nm. Pyrogene Kieselsäure wird beispielsweise unter der Bezeichnung CAB-O-SIL® vertrieben und ist ein bekanntes Verdickungsmittel für Mineralöl. Ein anderes Beispiel für einen geeigneten Gelbildner ist Bentonit. Auch das als Gelbildner bekannte Natriumcarbomer kann verwendet werden.

Eingesetzt wird der Gelbildner bevorzugt in einer Menge von 0 - 4,0 Gew.-%, weiter bevorzugt 0,1 - 2,0 Gew.-%, noch weiter bevorzugt 0,5 - 2,0 Gew.-%, bezogen auf das Gewicht der Klebeschicht.

In einer besonders bevorzugten Ausführungsform der erfindungsgemäßen TTS besteht die Klebeschicht zu 99,5 Gew.-% aus Mischungen von Polyisobutylen mit unterschiedlichen Molekulargewichten und Polybuten mit unterschiedlichen Molekulargewichten und zu 0,5 Gew.-% Gelbildner, bevorzugterweise hochdispersem Siliziumdioxid oder pyrogener Kieselsäure, bezogen auf das Gesamtgewicht der Klebeschicht.

*Deckschicht*

Die Deckschicht der erfindungsgemäßen TTS ist vorzugsweise okklusiv, also abschließend. Derartige Deckschichten können in bevorzugter Ausführungsform aus Polyolefinen, insbesondere Polyethylen, oder aus Polyester sowie Polyurethanen bestehen. Auch Schichten, die mehrere verschiedene Polymere übereinander angeordnet beinhalten, sind vorteilhafterweise einsetzbar. Geeignete Materialien umfassen Polyolefin, Zellophan, Celluloseacetat, Ethylcellulose, mit Weichmachern versehene Vinylacetat-Vinylchlorid Copolymere, Ethylen-Vinylacet Copolymere, Polyethylentherephtalat, Nylon, Polyethylen, Polypropylen, Polyvinylidenchlorid, Ethylen-Metacrylat Copolymere, Papier, das gegebenenfalls beschichtet sein kann, textile Gewebe, Aluminiumfolie und Polymer-Metall Kompositmaterialien. Besonders bevorzugt sind Polyesterfolien, wie Polyethylentherephtalatfolien. Die Dicke der Rückschicht kann, wie im Stand der Technik üblich, zum Beispiel 10 μm bis 100 μm, zum Beispiel etwa 40 μm (nominelle Dicke) betragen. Ganz besonders bevorzugt sind Verbundfolien aus pigmentiertem PE, PETP und Aluminium.

*Abziehschicht*

Auf der Klebeschicht befindet sich erfindungsgemäß eine Abziehschicht, auch als "release liner" bezeichnet.

Diese Abziehschicht ist bevorzugt aus polymerem Material hergestellt, das gegebenenfalls auch metallisiert sein kann. Beispiele für bevorzugt eingesetzte Materialien sind Polyurethane, Polyvinylacetat, Polyvinylidenchlorid, Polypropylen, Polycarbonat, Polystyrol, Polyethylen, Polyethylentherephtalat, Polybutylentherephtalat sowie gegebenenfalls mit entsprechenden Polymeren oberflächenbeschichtetes Papier. Bevorzugt handelt es sich hierbei um eine einseitig oder beidseitig fluoropolymerbeschichtete oder silikonisierte Abziehschicht. Besonders bevorzugt sind handelsübliche fluoropolymerbeschichtete oder silikonisierte Polyesterfolien, wie die einseitig silikonisierten Handelsprodukte Primeliner 100 μm und Perlasic LF 75 μm (Firma Loparex, NL und Perlen Converting AG, Schweiz) oder die einseitig fluoropolymerbeschichtete Produkte wie z.B. ScotchPak 1022 (3M Drug delivery).

*Weitere Aspekte und Ausführungsformen*

Eine besonders bevorzugte Ausführungsform der erfindungsgemäßen TTS ist ein TTS zur Verabreichung eines Wirkstoffes durch die Haut, umfassend:

a) eine Deckschicht,

b) ein auf der Deckschicht befindliche Wirkstoffschicht enthaltend 30 - 50 Gew.-% Wirkstoff und 50 - 70 % Polymermatrix, bezogen auf das Gesamtgewicht der Wirkstoffschicht, wobei die Polymermatrix im wesentlichen aus einem Acrylat-Polymer ohne Hydroxylgruppen und ohne Carboxylgruppen, oder im wesentlichen aus einem Acrylat-Copolymer ohne Hydroxylgruppen und ohne Carboxylgruppen besteht, und wobei der Wirkstoff Rivastigmin oder ein physiologisch verträgliches Salz, Hydrat, Solvat oder Derivat davon ist;

c) eine auf der Wirkstoffschicht befindliche, die Freisetzung des Rivastigmins kontrollierende Membran;

d) eine auf der Membran befindliche Klebeschicht bestehend aus 0 - 1 Gew.-% Siliziumdioxid und 99 - 100 Gew.-% eines Gemisches aus einem Polyisobutylenpolymer mit einem mittleren Molekulargewicht $M_V$ von etwa 40.000 g/mol, einem Polyisobutylenpolymer mit einem mittleren Molekulargewicht $M_V$ von etwa 400.000 g/mol, einem Polybutenpolymer mit einem mittleren Molekulargewicht $M_N$ von etwa 2.500 g/mol und einem Polybutenpolymer mit einem mittleren Molekulargewicht $M_N$ von etwa 6.000 g/mol; und

e) eine auf der Klebeschicht befindliche Abziehschicht.

Besonders bevorzugt ist die Menge an Antioxidationsmittel in der Gesamtformulierung dieser Ausführungsform ohne Deck- und Abziehschicht weniger als 0,1 Gew.-%, bevorzugt weniger als 0,01 %.

In einer besonderen Ausführungsform enthält das erfindungsgemäße TTS kein Antioxidationsmittel ausgewählt aus der Gruppe bestehend aus Vitamin E und Estern davon, Butyhydroxytoluol und Butylhydroxyanisol.

Vorzugsweise weist das erfindungsgemäße TTS ein im wesentlichen lineares Permeationsprofil auf. Die Permeation ist dabei im wesentlichen linear über einen Zeitraum von wenigstens 24 Stunden, vorzugsweise wenigstens 48 Stunden, bevorzugter wenigstens 72 Stunden, am bevorzugtesten wenigstens 96 Stunden. Die Permeation kann durch dem Fachmann an sich bekannte Permeationstests bestimmt werden, beispielsweise durch den in vitro-Permeationstest gemäß "OECD GUIDELINE FOR THE TESTING OF CHEMICALS. Skin Absorption: *in vitro* method." Test Guideline 428. Adopted 13 April 2004: 1-8.

Ein weiterer Aspekt der vorliegenden Erfindung ist die Bereitstellung eines Verfahrens zur Herstellung des erfindungsgemäßen TTS. Das Verfahren umfasst

i) die Herstellung einer die Wirkstoffschicht enthaltenden Komponente, enthaltend die Deckschicht und die Wirkstoffschicht, welches sich auf der Seite der Deckschicht befindet, welche als die der Haut zugewandte Seite bestimmt ist;

ii) die Herstellung einer die Klebeschicht enthaltenden Komponente, enthaltend die Abziehschicht und die sich auf der Abziehschicht befindliche Klebeschicht und eine die Freisetzung des Wirkstoffs kontrollierenden Membran;

iii) das aufeinander Laminieren der Komponenten aus i) und ii), so dass Deck- und Abziehschicht im Querschnitt des fertigen TTS die gegenüberliegenden, äußersten Schichten darstellen.

Eine Ausführungsform des Verfahrens umfasst:

i) das Auftragen und gegebenenfalls anschließende Trocknen eines Films einer die Wirkstoffschicht bildenden

Zusammensetzung, gegebenenfalls in Form einer Lösung oder Dispersion in einem geeigneten Medium, auf die Seite der Deckschicht, welche als die der Haut zugewandte Seite bestimmt ist, und gegebenenfalls das Kaschieren mit der die Freisetzung des Wirkstoffs kontrollierenden Membran; ;

ii) das Auftragen und gegebenenfalls anschließende Trocknen eines Films einer die Klebeschicht bildenden Zusammensetzung, gegebenenfalls in Form einer Lösung oder Dispersion in einem geeigneten Medium, auf die Abziehschicht und

iii) das aufeinander Laminieren der Komponenten aus i) und ii), so dass Deck-und Abziehschicht im Querschnitt des fertigen TTS die gegenüberliegenden, äußersten Schichten darstellen.

Eine andere Ausführungsform des Verfahrens umfasst:

i) das Auftragen und gegebenenfalls anschließende Trocknen eines Films einer die Wirkstoffschicht bildenden Zusammensetzung, gegebenenfalls in Form einer Lösung oder Dispersion in einem geeigneten Medium, auf eine silikonisierte Abziehfolie ("intermediate Liner") und das Kaschieren mit der Deckschicht;

ii) Abziehen der silikonisierten Abziehfolie ("intermediate Liner") und Zukaschieren der die Freisetzung des Wirkstoffs kontrollierenden Membran;

iii) das Auftragen und gegebenenfalls anschließende Trocknen eines Films einer die Klebeschicht bildenden Zusammensetzung, gegebenenfalls in Form einer Lösung oder Dispersion in einem geeigneten Medium, auf die Abziehschicht und

iv) das aufeinander Laminieren der Komponenten aus ii) und iii), so dass Deck- und Abziehschicht im Querschnitt des fertigen TTS die gegenüberliegenden, äußersten Schichten darstellen.

Die Herstellung eines bevorzugten TTS kann erfolgen, indem man zunächst die Komponenten für die Wirkstoffschicht, also den Wirkstoff (Rivastigmin) und das matrixbildende Polymer, bzw. Copolymer oder eine Mischung davon, in einem organischen Lösungsmittel wie Heptan oder Ethylacetat dispergiert, bzw. löst (sofern das Polymer nicht bereits gelöst vorliegt). Üblicherweise liegt das matrixbildende Polymer, bzw. Copolymer oder die Mischung davon bereits in einem Lösungsmittel vor. Dabei wird erfindungsgemäß ein Polymer und/oder Copolymer eingesetzt, das wie oben im Zusammenhang mit den erfindungsgemäßen TTS definiert ist, also ein Polymer und/oder Copolymer ohne Hydroxylgruppen und ohne Carboxylgruppen. Die oben als bevorzugt genannten Ausführungsformen der Polymermatrix gelten entsprechend für das erfindungsgemäße Verfahren. Bei der Herstellung der Wirkstoffschicht wird bevorzugt ein flüchtiges organisches Lösungsmittel eingesetzt. Diese Mischung wird dann als gleichmäßige Schicht auf der Deckschicht aufgebracht und getrocknet. Sofern eine die Freisetzung des Wirkstoffs kontrollierende Membran aufgebracht werden soll, kann diese, nachdem die Wirkstoffschichtgetrocknet ist, auf der der Deckschicht gegenüberliegenden Seite der Wirkstoffschicht aufgebracht werden, anstelle des "intermediate liners". Vorzugsweise wird die Komponente für die Wirkstoffschicht zum Schutz mit einer Folie, bevorzugt eine silikonisierte Polyesterfolie, auch als "intermediate liner" bezeichnet, versehen, welche auf der der Deckschicht gegenüberliegenden Seite der Wirkstoffschicht aufgebracht wird. Alternativ, bzw. hierzu äquivalent kann die Mischung auch zunächst auf den "intermediate liner" aufgetragen und getrocknet werden, wobei die Deckschicht im Anschluss auf die dem "intermediate liner" gegenüberliegenden Seite die Wirkstoffschicht aufgebracht wird. Der "intermediate liner" wird entfernt, kurz bevor die Wirkstoffschicht komponente mit der die Klebeschicht enthaltenden Komponente zusammengefügt wird. Gegebenenfalls kann nach Abziehen des "intermediate liners" auch die Kontrollmembran zukaschiert werden, bevor die Zusammenfügung mit der Klebeschicht erfolgt.

In einem separaten Arbeitsschritt wird die Klebeschicht hergestellt, indem das den Haftkleber bildende (in organischem Lösungsmittel gelöste) Polymerengemisch, bevorzugt Polyisobutylen von unterschiedlichem mittleren Molekulargewicht, gegebenenfalls zusammen mit dem Klebkraftverstärker, Gelbildner und/oder dem Weichmacher in einem organischen Lösungsmittel wie Heptan dispergiert wird. Bevorzugt werden jedoch der Haftkleber und der Klebkraftverstärker und/oder Weichmacher in dem organischen Lösungsmittel gelöst und anschließend der Gelbildner in dieser Lösung dispergiert. Diese Mischung wird dann auf die Abziehfolie aufgebracht und trocknen gelassen. Die oben als bevorzugt genannten Ausführungsformen der Klebeschicht gelten entsprechend für das erfindungsgemäße Verfahren.

Die in den beiden Verfahrensschritten erhaltenen Komponenten werden anschließend miteinander laminiert, und zwar bevorzugt so, dass die Klebeschicht direkt auf die Wirkstoffschicht aufgebracht wird. In denjenigen Ausführungsformen, in denen eine Membran eingesetzt wird, wird die Klebeschicht auf die Membran aufgebracht. Anschließend können aus der fertigen laminierten Folie Stücke gewünschter Größe ausgestanzt und verpackt werden.

Bei den einzelnen Verfahrensschritten werden die organischen Lösungsmittel, die erforderlich sind, um die jeweiligen Komponenten in Lösung zu bringen bzw. zu dispergieren, dadurch entfernt, dass die Produkte ansteigenden Temperaturen, gegebenenfalls auch unter Anwendung eines Unterdrucks, ausgesetzt werden.

Einen weiteren Aspekt der vorliegenden Offenbarung stellt die Verwendung eines Polymers oder Copolymers, das weder Aminogruppen noch Hydroxylgruppen noch Carboxylgruppen aufweist, in einem TTS enthaltend Rivastigmin, welches für einen Anwendungszeitraum von mindestens zwei oder mindestens drei Tagen geeignet ist, dar. Bevorzugt ist die Verwendung von Polyacrylaten, Acrylat-Vinylacetat Copolymeren, Polyisobutylen und Styrol-Butadien Copolymeren wie oben definiert. Erfindungsgemäß stellen diese Polymere oder Copolymere die Polymermatrix der Wirkstoffschicht dar, in die der Wirkstoff Rivastigmin eingebettet ist.

Ein weiterer Aspekt der Offenbarung ist die Verwendung eines Polymers oder Copolymers, das keine freien Hydroxylgruppen und keine freien Carboxylgruppen aufweist, zur Stabilisierung von Rivastigmin in einem TTS, oder zur Verringerung des Abbaus von Rivastigmin in einem TTS. Ein weiterer Aspekt der Offenbarung ist die Verwendung eines Polymers oder Copolymers, das weder freie Aminogruppen noch freie Hydroxylgruppen noch freie Carboxylgruppen aufweist, zur Stabilisierung von Rivastigmin in einem TTS, oder zur Verringerung des Abbaus von Rivastigmin in einem TTS.

In der offenbarten Verwendung kommen bevorzugt aminogruppenfreie, hydroxylgruppenfreie und carboxylgruppenfreie Polyacrylate und Polyacrylatcopolymere, wie Acrylat-Vinylacetat Copolymere zum Einsatz.

In einer besonders bevorzugten Ausführungsform der offenbarten Verwendung wird das Acrylat-Vinylacetat Copolymer Duro-Tak® 87-4098 verwendet.

In einer weiteren, besonders bevorzugten Ausführungsform der offenbarten Verwendung wird das Acrylatpolymer Duro-Tak® 87-9088 verwendet.

Einen weiteren Aspekt der vorliegenden Offenbarung stellt die Verwendung von Polyisobutylen und Polybuten als ausschließliche Bestandteile des Haftklebers/Klebkraftverbesserers in der Klebeschicht eines TTS dar, welches für einen Anwendungszeitraum von mindestens zwei oder mindestens drei Tagen geeignet ist. Polyisobutylen und Polybuten liegen dabei gemäß den obenstehenden Aspekten der vorliegenden Erfindung bevorzugt jeweils als Mischungen unterschiedlicher mittlerer Molekulargewichte vor.

Ein weiterer Aspekt der vorliegenden Erfindung ist die Bereitstellung der erfindungsgemäßen TTS zur Behandlung von Alzheimer und Parkinsondemenz. Das erfindungsgemäße TTS wird dabei bevorzugt für einen Anwendungszeitraum von mindestens zwei oder mindestens drei Tagen hergerichtet. Längere Anwendungszeiträume sind ebenso möglich.

Im Folgenden werden bevorzugte Ausführungsformen des erfindungsgemäßen TTS anhand von experimentellen Beispielen beschrieben und deren Eigenschaften hinsichtlich der Stabilität bestimmt.

**<u>Erläuterungen der Figuren 1-7:</u>**

Im Folgenden bezeichnet der Parameter "n" die Anzahl der Wiederholungen der durchgeführten Messungen, welche zu gemittelten Ergebnissen führen.

<u>Figur 1:</u> Schematischer Querschnitt eines Rivastigmin TTS mit Membran (nicht maßstabsgerecht).

<u>Figur 2:</u> Diagramm bezüglich des maximalen kalten Fluss (Cold Flow) der Wirkstoffschichten nach 9-wöchiger Lagerung bei 25 °C/60% r.F. und 40 °C/75% r.F. (n = 2).

<u>Figur 3:</u> Diagramm bezüglich der Klebkraft der gemäß den Beispielen hergestellten Klebeschichten (n = 3).

<u>Figur 4:</u> In vitro-Permeationsprofil von Rivastigmin aus Exelon® (n = 6).

<u>Figur 5:</u> In vitro-Permeationsprofile von Rivastigmin aus Zweischichtlaminaten mit und ohne Membran (n = 4).

<u>Figur 6:</u> *In* vitro-Permeationsprofile von Rivastigmin aus Zweischichtlaminaten mit Klebeschichten basierend auf einem Polyacrylat-Kleber (n = 4).

<u>Figur 7:</u> In vitro-Permeationsprofile von Rivastigmin aus Zweischichtlaminaten mit verschiedenen Membranen und Klebeschichten im Vergleich zum Exelon® (n ≥ 4).

**<u>Beispiele</u>**

Die in den nachfolgenden Formulierungsbeispielen eingesetzten Komponenten können folgendermaßen näher

beschrieben werden:

Tabelle 1: Übersicht der Komponenten der Formulierungsbeispiele

| Komponentenbezeichnung | Chemische Beschreibung | Funktion |
|---|---|---|
| Duro-Tak® 87-4098 | Acrylat/Vinylacetat Copolymer | Matrixpolymer |
| Duro-Tak® 87-9088 | Acrylat Copolymer | Matrixpolymer |
| Cab-O-Sil® | Pyrogenes Siliziumdioxid | Gelbildner |
| Oppanol® B10 | Polyisobutylen ($M_V$= ca. 4x10$^4$ g/mol) | Haftkleber |
| Oppanol® B50 SF | Polyisobutylen ($M_V$= ca. 4x10$^5$ g/mol) | Haftkleber |
| Indopol H-100, H-1900, H-6.000, H-18.000 | Polybuten ($M_N$ = 910, 2.500, 4.200 bzw. 6.000 g/mol) | Klebrigmacher |
| Eudragit® E100 | Acrylat Copolymer | Matrixpolymer |

**Beispiel 1**

Herstellung verschiedener Wirkstoffschicht-Formulierungen

Es wurden drei unterschiedliche Wirkstoffschicht-Formulierungen, welche Rivastigmin Base enthalten, hergestellt. Eine Übersicht der Bestandteile der verschiedenen Formulierungen ist in Tabelle 2 wiedergegeben.

Tabelle 2: Verwendete Wirkstoffschichten

| Charge | Wirkstoffschicht (R) | Matrixgewicht mg/10cm$^2$ |
|---|---|---|
| 010RIDTDS | 40 % Rivastigmin; 60 % DT® 87-9088 | 64 |
| 011RIDTDS | 40 % Rivastigmin; 60 % DT® 87-4098 | 62 |
| 012RIDTDS | 40 % Rivastigmin; 60 % Eudragit® E100 | 54 |

In Figur 2 sind die Ergebnisse zur physikalischen Stabilität (Cold flow) der obenstehenden Rerservoir-Formulierungen wiedergegeben. Die Formulierung mit DT® 87-9088 (010RIDTDS) zeigt nach 9-wöchiger Lagerung den geringsten Cold flow und eignet sich daher besonders als Matrixbildner für die Wirkstoffschicht.

**Beispiel 2**

Es wurden sechs unterschiedliche Formulierungen der Klebeschicht hergestellt. Eine Übersicht der Bestandteile der verschiedene Formulierungen ist in Tabelle 3 wiedergeben.

Tabelle 3: Verwendete Klebeschichten

| Charge | Klebeschicht (A) | Matrixschicht mg/10 cm$^2$ |
|---|---|---|
| 019RIDTDS | 17,91 % Oppanol® B10 SFN; 31,84 % Oppanol® B50SF; 29,85 % Indopol® H-100; 19,90 % Indopol® H-1900; 0,50 % Cab-O-Sil® M5P | 30 |
| 005RIDTDS | 24,88 % Oppanol® B10 SFN; 24,88 % Oppanol® B50SF; 24,88 % Indopol® H-100; 24,88 % Indopol® H-1900; 0,50 % Cab-O-Sil® M5P | 32 |
| 013RIDTDS | 24,88 % Oppanol® B10 SFN; 24,88 % Oppanol® B50SF; 24,88 % Indopol® H-100; 24,88 % Indopol® H-6000; 0,50 % Cab-O-Sil® M5P | 32 |
| 014RIDTDS | 24,88 % Oppanol® B10 SFN; 24,88 % Oppanol® B50SF; 24,88 % Indopol® H-100; 24,88 % Indopol® H-18000; 0,50 % Cab-O-Sil® M5P | 30 |
| 015RIDTDS | 29,85 % Oppanol® B10 SFN; 19,90 % Oppanol® B50SF; 19,90 % Indopol® H-1900; 29,85 % Indopol® H-18000; 0,50 % Cab-O-Sil® M5P | 35 |
| C006RIVTDS | 64,5 % Oppanol® B10 SFN/B50SF (4/6); 35,0 % Paraffin; 0,5 % Cabo-O-Sil® | 30 |

In Figur 3 sind die Ergebnisse von Messungen der Klebkraft der hergestellten Klebeschichten abgebildet.

Daraus geht hervor, dass durch Zusatz von Indopol® zum Oppanol®-Kleber die Klebkraft erheblich verbessert wird, verglichen mit der Verwendung von Paraffin. Darüber hinaus kann die Klebkraft durch die gezielte Auswahl der Molekulargewichte der eingesetzten Polymere weiter erhöht und gesteuert werden.

**Beispiel 3**

Schließlich wurden sechs unterschiedliche Chargen von TTS-Formulierungen hergestellt. Eine Übersicht über die Zusammensetzung der verschiedenen Chargen ist in Tabelle 4 zusammengestellt.

Tabelle 4: Verwendete Chargen:

| **Charge** | **Wirkstoffschicht (R)** | **Klebeschicht (A)** | **Membran** | **Matrixgewicht mg/10 cm$^2$** |
|---|---|---|---|---|
| 015/017 RIDTDS ohne Membran | 40% Rivastigmin; 60% DT® 87-9088 | 29,85% Oppanol® B10 SFN<br>19,90% Oppanol® B50SF<br>19,90% Indopol® H-1900<br>29,85% Indopol® H-18000<br>0,50% Cap-O-Sil M5P | --- | 015RIDTDS (A): 35<br>017RIDTDS (R): 60 |
| 015/017 RIDTDS CoTran 9719 | 40% Rivastigmin; 60% DT® 87-9088 | 29,85% Oppanol® B10 SFN<br>19,90% Oppanol® B50SF<br>19,90% Indopol® H-1900<br>28,85% Indopol® H-18000<br>0,50% Cap-O-Sil M5P | CoTran™ 9719 | 015RIDTDS (A): 35<br>017RIDTDS (R): 60 |
| 009/010 RIDTDS Celgard 2400 | 40% Rivastigmin; 60% DT® 87-9088 | 10% Rivastigmin; 90% DT® 87-2516 | Celgard® 2400 | 009RIDTDS (A): 33<br>010RIDTDS (R): 64 |
| 009/010 RIDTDS Solupor 10P05A | 40% Rivastigmin; 60% DT® 87-9088 | 10% Rivastigmin; 90% DT® 87-2516 | Solupor® 10P05A | 009RIDTDS (A): 33<br>010RIDTDS (R): 64 |
| 029/030 RIDTDS Solupor 10P05A | 40% Rivastigmin; 60% DT® 87-9088 | 20% Oppanol® B10 SFN<br>30% Oppanol® B50SF<br>30% Indopol® H-100<br>20% Indopol® H-1900 | Solupor® 10P05A | 029RIDTDS (A): 34<br>030RIDTDS (R): 61 |
| 028/030 RIDTDS CoTran 9719 | 40% Rivastigmin; 60% DT® 87-9088 | 30% Oppanol® B10 SFN<br>20% Oppanol® B50SF<br>20% Indopol® H-1900<br>30% Indopol® H-18000 | CoTran™ 9719 | 028RIDTDS (A): 28<br>030RIDTDS<br>(R): 61 |

Herstellungsverfahren

1. Herstellung der Wirkstoffschicht

Der Acrylat Kleber wurde vorgelegt und Rivastigmin und Ethylacetat dazu gewogen. Anschliessend wurden die Komponenten in ausreichend Ethylacetat mittels eines Rührers gemischt, sodass eine streichfähige, homogene Beschichtungsmasse entsteht.

Die homogene Beschichtungsmasse wurde auf einer silikonisierten Folie ("intermediate liner") als dünner Film aufgetragen. Der Matrixfilm wurde bei 60°C/20 min und 80°C/5 min getrocknet und anschliessend mit einer Deckschicht aus PET kaschiert.

Anschließend wurde der "intermediate liner" abgezogen und die Kontrollmembran zukaschiert.

2. Herstellung der Adhäsivschicht und des Gesamtlaminats

Die Polyisobutylenkleber wurden zusammen eingewogen und gemischt. Anschliessend wurden unter Rühren Heptan und Cab-O-Sil® zugegeben und so lange gerührt bis die Masse homogen war.

Die Masse wurde auf eine Abziehschicht ("release liner") als dünner Film aufgetragen und anschliessend werden die Lösemittel bei 60°C/20 min und 80°C/5 min abgezogen. Nach der Trocknung wird das Laminat mit der Wirkstoffschichtkaschiert.
Aus dem erhaltenen Laminat wurden Pflaster geeigneter Grösse ausgestanzt.

Mit den Chargen aus Tabelle 4 und dem kommerziellen Produkt Exelon® TDS, einer Eintagesformulierung, wurden in vitro-Maus-Hautpermeationstests durchgeführt.

Die Ergebnisse dieser Tests sind in den Figuren 4 bis 7 wiedergegeben. Figur 4 zeigt das Permeationsprofil von Rivastigmin aus dem kommerziell verfügbaren Exelon® TDS. Durch den Kurvenverlauf in Figur 4 wird deutlich, dass die Abgaberate von Rivastigmin bereits nach 24 Stunden deutlich abnimmt. Dieses System gewährleistet daher keine kontinuierliche und gleichmäßige Wirkstoffabgabe über einen Anwendungszeitraum von mehr als 24 Stunden.

Aus Figur 5 geht hervor, dass nur durch die zwischenliegende Membran eine mehrtägige kontinuierliche und gleichmäßige Wirkstoffabgabe ermöglicht wird.

Die Formulierungsansätze 009/010RIDTDS Celgard® 2400 und 009/010RIDTDS Solupor® 10P05A, die Polyacrylat als Matrixbildner für die Klebeschicht enthalten, wiesen überraschenderweise eine im Vergleich zum Exelon® TDS ähnliche Kinetik auf (vgl. Figur 4 und 6). Daraus geht hervor, dass sich Polymere aus der Gruppe der Polyacrylate und dessen Copolymere nicht als Matrixbildner für die Klebeschicht eignen, da sie, selbst bei zwischenliegender Membran, keine ausreichende Steuerung der Wirkstoffabgabe über die mehrtägige Applikationszeit gewährleisten.

Aus den Figuren 5 und 7 geht hervor, dass durch die Kombination einer die Freisetzung des Wirkstoffs kontrollierenden Membran und einer Klebeschicht basierend auf einem Polyisobutylen/Polybuten-Gemisch eine Steuerung der Arzneistoffabgabe über ein mehrtägiges Zeitintervall ermöglichen

**Beispiel 4: Stabilitätstest**

Nach dem oben angegebenen Verfahren wurden noch zwei weitere Formulierungen hergestellt:

Tabelle 5

| | **Formulierung I (060/062RIDTDS_Cotran9719)** | **Formulierung II (058/062RIDTDS_Cotran9728)** |
|---|---|---|
| **Wirkstoffschicht 60 g/m²** | Rivastigmin (40%)<br>DT® 87-9088 (60%) | Rivastigmin (40%)<br>DT® 87-9088 (60%) |
| **Membran** | CoTran™ 9719 | CoTran™ 9728 |
| **Adhäsivschicht 30 g/m²** | Oppanol® B10 SFN (30%)<br>Oppanol® B50 SF (20%)<br>Indopol® H-1900 (20%)<br>Indopol® H-18000 (30%) | Oppanol® B10 SFN (30%)<br>Oppanol® B50 SF (40%)<br>Indopol® H-1900 (30%) |

Die ausgestanzten TTS wurden in Beutel aus Aluminium-Verbundfolien versiegelt und jeweils mindestens einen Monat lang bei 25°C und 60 % relativer Luftfeuchtigkeit, beziehungsweise bei 40°C und 75 % relativer Luftfeuchtigkeit gelagert. Anschließend wurde der Gehalt eventuell entstandener Verunreinigungen als Folge der Zersetzung von

Rivastigmin mittels HPLC und UV-Absorption ermittelt.

Die erfindungsgemäßen TTS zeigten eine ausgezeichnete Stabilität über mehrere Monate. Nach Lagerung wurden nur sehr geringe Mengen an Verunreinigungen/ Zersetzungsprodukten nachgewiesen, obwohl die Wirkstoffschicht keine Antioxidantien enthielt. Dies wurde durch den Einsatz einer Polymermatrix ohne Hydroxylgruppen und ohne Carboxylgruppen erreicht.

Die Ergebnisse sind in der folgenden Tabelle 6 zusammengefasst:

Tabelle 6: Verunreinigungen nach Lagerung

| | | **Temperatur** | **Initial** | **1 Monat** | **2 Monate** | **3 Monate** |
|---|---|---|---|---|---|---|
| | **Charge** | **°C/% .F** | | | | |
| Reinheit n=3 | 060/062RIDTDS _CoTran9719 | 25 / 60 | **Imp.** 1: 0,04% **(<RL)**; **Imp.** 4: 0,02% **(<RL)**; **Imp. 5**: 0,05% **(<RL)**; → Summe: 0,0% | **Imp. 1:** 0,05% **(< RL);**<br>**Imp. 4:** 0,06% (< RL);<br>**Imp. 5**: 0,14%<br>→ Summe: 0,14% | **Imp. 1:** 0,02% **(< RL);**<br>**Imp.** 4: 0,07% **(< RL);**<br>**Imp. 5**: 0,18%<br>→ Summe: 0,18% | **Imp. 1**: 0,02% **(< RL);**<br>**RRT=0,84**: 0,01% **(<RL)**;<br>**Imp.** 2: 0,02% **(< RL);**<br>**Imp.** 4: 0,06% **(<RL)**;<br>**Imp. 5: 0,17%**<br>→ Summe: 0,17% |
| | | 40 / 75 | | **Imp. 1:** 0,04% **(< RL);**<br>**RRT=0,85:** 0,06% (< RL);<br>**Imp. 2:** 0,02% **(< RL);**<br>**Imp. 4: 0,11%;**<br>**Imp. 5: 0,23%**<br>→ Summe: 0,34% | | |
| | 058/062RIDTDS _CoTran9728 | 25 / 60 | **Imp. 1:** 0,04% (**<RL**); **Imp. 4**: 0,02% **(<RL)**; **Imp. 5:** 0,05% **(< RL);** | **Imp. 1:** 0,05% **(< RL);**<br>**Imp. 4:** 0,05% (< **RL**);<br>**Imp. 5**: 0,15%<br>→ Summe: 0,15% | **Imp. 1**: 0,02% (**<RL**);<br>**Imp.** 4: 0,06% **(< RL);**<br>**Imp. 5: 0,18%**<br>→ Summe: 0,18% | **Imp. 1:** 0.02% (**< RL**);<br>**RRT=0,84:** 0,02% **(<RL);**<br>**Imp. 2:** 0,02% **(< RL);**<br>**Imp. 4:** 0,09% **(<RL);**<br>**Imp. 5: 0,18%;**<br>→ Summe: 0,18% |

(fortgesetzt)

| | | Temperatur | Initial | 1 Monat | 2 Monate | 3 Monate |
|---|---|---|---|---|---|---|
| | Charge | °C/% .F | | | | |
| | | 40 / 75 | → Summe: 0,0% | **Imp. 1:** 0,05% **(< RL); RRT=0,85:** 0,04% (< RL); **Imp. 2**: 0.03% **(< RL)**; **Imp. 4:** 0,09% **(< RL); Imp. 5: 0,22%** → Summe: 0,22% | | |
| RL="Reporting Limit" (Nachweisgrenze) RRT=relative Retentionszeit (HPLC) | | | | | | |

**Patentansprüche**

1. Transdermales therapeutisches System zur Verabreichung eines Wirkstoffs durch die Haut umfassend die zueinander in der folgenden Reihenfolge angeordneten Schichten:

   a) eine Deckschicht;
   b) eine Wirkstoffschicht umfassend eine den Wirkstoff enthaltende Polymermatrix;
   c) eine die Freisetzung des Wirkstoffs kontrollierende Membran;
   d) eine Klebeschicht umfassend einen Haftkleber, der ein Polyisobutylen oder ein Gemisch von mehreren Polyisobutylenen enthält; und
   e) eine Abziehschicht;

   wobei der Wirkstoff Rivastigmin oder ein physiologisch verträgliches Salz, Hydrat oder Solvat davon ist, **dadurch gekennzeichnet, dass** das Polymer/die Polymere der Polymermatrix keine freien Hydroxylgruppen und keine freien Carboxylgruppen enthält/enthalten, und dass die Klebeschicht weiterhin ein Polybuten oder ein Gemisch von mehreren Polybutenen enthält.

2. Transdermales therapeutisches System nach Anspruch 1, **dadurch gekennzeichnet, dass** es keine Tocopherole enthält.

3. Transdermales therapeutisches System nach Anspruch 1 oder 2, **dadurch gekennzeichnet, dass** es kein Antioxidationsmittel enthält, das ausgewählt ist aus der Gruppe bestehend aus Tocopherolen, Butylhydroxdyanisol und Butylhydroxytoluol.

4. Transdermales therapeutisches System nach einem der Ansprüche 1 bis 3, **dadurch gekennzeichnet, dass** es kein Antioxidationsmittel enthält.

5. Transdermales therapeutisches System nach einem der Ansprüche 1 bis 4, wobei die Klebeschicht 98-100 Gew.-% Haftkleber einschließlich Polybuten, und 0 - 2,0 Gew.-% Gelbildner, bezogen auf das Gesamtgewicht der Klebeschicht, enthält.

6. Transdermales therapeutisches System nach einem der Ansprüche 1 bis 5, zur Anwendung über einen Zeitraum von mindestens zwei Tagen.

7. Transdermales therapeutisches System nach einem der Ansprüche 1 bis 6, **dadurch gekennzeichnet, dass** der Haftkleber aus mindestens zwei Polyisobutylenen mit unterschiedlichen mittleren Molekulargewichten und mindestens zwei Polybutenen mit unterschiedlichen mittleren Molekulargewichten besteht.

**8.** Transdermales therapeutisches System nach Anspruch 7, **dadurch gekennzeichnet, dass** das erste Polyisobutylenpolymer ein mittleres Molekulargewicht $M_v$ von etwa 40.000 g/mol hat, und das zweite Polyisobutylenpolymer ein mittleres Molekulargewicht $M_v$ von etwa 400.000 g/mol hat.

**9.** Transdermales therapeutisches System nach einem der Ansprüche 7 bis 8, **dadurch gekennzeichnet, dass** das erste Polybutenpolymer ein mittleres Molekulargewicht $M_n$ im Bereich von 700 - 2.800 g/mol hat, und das zweite Polybutenpolymer ein mittleres Molekulargewicht $M_n$ im Bereich von 2.200 - 6.500 g/mol hat.

**10.** Transdermales therapeutisches System nach einem der Ansprüche 1 bis 9, wobei die Wirkstoffschicht 30 - 50 Gew.-% Wirkstoff und 50 - 70 Gew.-% Polymermatrix, bezogen auf das Gesamtgewicht der Wirkstoffschicht, enthält.

**11.** Transdermales therapeutisches System nach einem der Ansprüche 1 bis 10, wobei die Polymermatrix der Wirkstoffschicht wenigstens ein Polymer und/oder Copolymer ohne freie Hydroxylgruppen und ohne freie Carboxylgruppen umfasst, ausgewählt aus der Gruppe bestehend aus Polyacrylaten, Acrylat-Vinylacetat Copolymeren, Polyisobutylen, Styrol-Butadien Copolymeren und Mischungen davon.

**12.** Transdermales therapeutisches System nach einem der Ansprüche 1 bis 11, **dadurch gekennzeichnet, dass** das TTS über einen Zeitraum von wenigstens 48 Stunden ein im wesentlichen lineares Permeationsprofil des Wirkstoffs zeigt.

**13.** Verfahren zur Herstellung eines transdermalen Systems nach einem der Ansprüche 1 bis 12, umfassend

i) die Herstellung einer die Wirkstoffschicht enthaltenden Komponente, enthaltend die Deckschicht und die Wirkstoffschicht, welches sich auf der Seite der Deckschicht befindet, welche als die der Haut zugewandten Seite bestimmt ist;
ii) die Herstellung einer die Klebeschicht enthaltenden Komponente, enthaltend die Abziehschicht und die sich auf der Abziehschicht befindliche Klebeschicht und eine die Freisetzung des Wirkstoffs kontrollierenden Membran;
iii) das aufeinander Laminieren der Komponenten aus i) und ii), so dass Deck- und Abziehschicht im Querschnitt des fertigen TTS die gegenüberliegenden, äußersten Schichten darstellen.

**14.** Verfahren nach Anspruch 13, umfassend

i) das Auftragen und gegebenenfalls anschließende Trocknen eines Films einer die Wirkstoffschicht bildenden Zusammensetzung, gegebenenfalls in Form einer Lösung oder Dispersion in einem geeigneten Medium, auf die Seite der Deckschicht, welche als die der Haut zugewandte Seite bestimmt ist, und gegebenenfalls das Kaschieren mit der die Freisetzung des Wirkstoffs kontrollierenden Membran;
ii) das Auftragen und gegebenenfalls anschließende Trocknen eines Films einer die Klebeschicht bildenden Zusammensetzung, gegebenenfalls in Form einer Lösung oder Dispersion in einem geeigneten Medium, auf die Abziehschicht und
iii) das aufeinander Laminieren der Komponenten aus i) und ii), so dass Deck-und Abziehschicht im Querschnitt des fertigen TTS die gegenüberliegenden, äußersten Schichten darstellen;

oder

i) das Auftragen und gegebenenfalls anschließende Trocknen eines Films einer die Wirkstoffschicht bildenden Zusammensetzung, gegebenenfalls in Form einer Lösung oder Dispersion in einem geeigneten Medium, auf eine silikonisierte oder fluoropolymerbeschichtete Abziehfolie ("intermediate Liner") und das Kaschieren mit der Deckschicht;
ii) Abziehen der silikonisierten Abziehfolie ("intermediate Liner") und Zukaschieren der die Freisetzung des Wirkstoffs kontrollierenden Membran;
iii) das Auftragen und gegebenenfalls anschließende Trocknen eines Films einer die Klebeschicht bildenden Zusammensetzung, gegebenenfalls in Form einer Lösung oder Dispersion in einem geeigneten Medium, auf die Abziehschicht und
iv) das aufeinander Laminieren der Komponenten aus ii) und iii), so dass Deck- und Abziehschicht im Querschnitt des fertigen TTS die gegenüberliegenden, äußersten Schichten darstellen.

**15.** Transdermales therapeutisches System nach einem der Ansprüche 1 bis 14 zur Verwendung in der Behandlung

von Alzheimer und Parkinsondemenz.

## Claims

1. A transdermal therapeutic system for administering an active substance through the skin comprising the layers arranged in the following order with respect to each other:

   a) a cover layer;
   b) an active substance layer comprising a polymer matrix containing the active substance;
   c) a membrane controlling the release of the active substance;
   d) an adhesive layer comprising a contact adhesive containing a polyisobutylene or a mixture of several polyisobutylenes; and
   e) a pull-off layer;

   wherein the active substance is rivastigmine or a physiologically compatible salt, hydrate or solvate thereof, **characterized in that** the polymer(s) of the polymer matrix contain(s) no free hydroxyl groups and no free carboxyl groups and **in that** the adhesive layer further contains a polybutene or a mixture of several polybutenes.

2. The transdermal therapeutic system according to claim 1, **characterized in that** it does not contain any tocopherols.

3. The transdermal therapeutic system according to any one of claims 1 or 2, **characterized in that** it does not contain any antioxidant selected from the group consisting of tocopherols, butylated hydroxyanisole, and butylated hydroxytoluene.

4. The transdermal therapeutic system according to any one of claims 1 to 3, **characterized in that** it does not contain any antioxidant.

5. The transdermal therapeutic system according to any one of claims 1 to 4, wherein the adhesive layer contains 98-100% by weight of the contact adhesive including polybutene, and 0-2.0% by weight of a gel-forming agent, based on the total weight of the adhesive layer.

6. The transdermal therapeutic system according to any one of claims 1 to 5 for application over a period of at least two days.

7. The transdermal therapeutic system according to any one of claims 1 to 6, **characterized in that** the contact adhesive consists of at least two polyisobutylenes with different average molecular weights and at least two polybutenes with different average molecular weights.

8. The transdermal therapeutic system according to claim 7, **characterized in that** the first polyisobutylene polymer has an average molecular weight $M_v$ of about 40,000 g/mol and the second polyisobutylene polymer has an average molecular weight $M_v$ of about 400,000 g/mol.

9. The transdermal therapeutic system according to any one of claims 7 to 8, **characterized in that** the first polybutene polymer has an average molecular weight $M_n$ in the range of 700-2,800 g/mol and the second polybutene polymer has an average molecular weight $M_n$ in the range of 2,200-6,500 g/mol.

10. The transdermal therapeutic system according to any one of claims 1 to 9, wherein the active substance layer contains 30-50% by weight of the active substance and 50-70% by weight of the polymer matrix, based on the total weight of the active substance layer.

11. The transdermal therapeutic system according to any one of claims 1 to 10, wherein the polymer matrix of the active substance layer comprises at least one polymer and/or copolymer without free hydroxyl groups and without free carboxyl groups selected from the group consisting of polyacrylates, acrylate-vinyl acetate copolymers, polyisobutylene, styrene-butadiene copolymers, and mixtures thereof.

12. The transdermal therapeutic system according to any one of claims 1 to 11, **characterized in that** the TTS shows a substantially linear permeation profile of the active substance over a period of at least 48 hours.

**13.** A method for the preparation of a transdermal system according to any one of claims 1 to 12 comprising

i) the preparation of a component containing the active substance layer that contains the cover layer and the active substance layer, which is on the side of the cover layer that is supposed to be the side facing the skin;
ii) the preparation of a component containing the adhesive layer that contains the pull-off layer and the adhesive layer on the pull-off layer and a membrane controlling the release of the active substance;
iii) laminating the components of i) and ii) onto each other such that the cover and pull-off layer in the cross-section of the finished TTS represent the opposing outermost layers.

**14.** The method according to claim 13 comprising

i) applying and optionally subsequent drying of a film of a composition forming the active substance layer, optionally in the form of a solution or dispersion in a suitable medium, onto the side of the cover layer supposed to be the side facing the skin, and optionally backing with the membrane controlling the release of the active substance;
ii) applying and optionally subsequent drying of a film of a composition forming the adhesive layer, optionally in the form of a solution or dispersion in a suitable medium, onto the pull-off layer; and
iii) laminating the components of i) and ii) onto each other such that the cover and pull-off layer in the cross-section of the finished TTS represent the opposing outermost layers;

or

i) applying and optionally subsequent drying of a film of a composition forming the active substance layer, optionally in the form of a solution or dispersion in a suitable medium, onto a siliconized or fluoro polymer-coated pull-off foil ("intermediate liner") and backing with the cover layer;
ii) pulling off the siliconized pull-off foil ("intermediate liner") and backing with the membrane controlling the release of the active substance;
iii) applying and optionally subsequent drying of a film of a composition forming the adhesive layer, optionally in the form of a solution or dispersion in a suitable medium, onto the pull-off layer; and
iv) laminating the components of ii) and iii) onto each other such that the cover and pull-off layer in the cross-section of the finished TTS represent the opposing outermost layers.

**15.** The transdermal therapeutic system according to any one of claims 1 to 14 for the use in the treatment of the Alzheimer’s disease and Parkinson’s dementia.

**Revendications**

**1.** Système thérapeutique transdermique pour l’administration d’une substance active par la peau, comprenant les couches disposées les unes par rapport aux autres dans l’ordre suivant :

a) une couche de recouvrement
b) une couche de substance active comprenant une matrice polymère contenant une substance active ;
c) une membrane contrôlant la libération de la substance active ;
d) une couche adhésive comprenant un adhésif de contact qui contient un polyisobutylène ou un mélange de plusieurs polyisobutylènes ; et
e) une couche pelliculable ;
dans lequel la substance active est la rivastigmine ou un sel, un hydrate ou un solvate physiologiquement acceptable de celle-ci, **caractérisé en ce que** le/les polymère(s) de la matrice de polymère ne contient/contiennent pas de groupes hydroxyle libres ni de groupes carboxyle libres, et **en ce que** la couche adhésive contient en outre un polybutène ou un mélange de plusieurs polybutènes.

**2.** Système thérapeutique transdermique selon la revendication 1, **caractérisé en ce qu’**il ne contient pas de tocophérols.

**3.** Système thérapeutique transdermique selon la revendication 1 ou 2, **caractérisé en ce qu’**il ne contient pas d’antioxydant qui est choisi dans le groupe comprenant les tocophérols, le butylhydroxyanisol et le butylhydroxytoluène.

**4.** Système thérapeutique transdermique selon l'une des revendications 1 à 3, **caractérisé en ce qu'**il ne contient pas d'antioxydants.

**5.** Système thérapeutique transdermique selon l'une des revendications 1 à 4, dans lequel la couche adhésive contient 98 à 100% en poids d'adhésif de contact comprenant du polybutène et 0 à 2,0% en poids d'agent gélifiant, par rapport au poids total de la couche adhésive.

**6.** Système thérapeutique transdermique selon l'une des revendications 1 à 5, pour son utilisation sur une durée d'au moins deux jours.

**7.** Système thérapeutique transdermique selon l'une des revendications 1 à 6, **caractérisé en ce que** l'adhésif de contact est constitué d'au moins deux polyisobutylènes présentant des poids moléculaires moyens différents et au moins deux polybutènes comportant des poids moléculaires moyens différents.

**8.** Système thérapeutique transdermique selon la revendication 7, **caractérisé en ce que** le premier polymère polyisobutylène a un poids moléculaire moyen $M_v$ d'environ 40 000 g/mole et le deuxième polymère polyisobutylène a un poids moléculaire moyen $M_v$ d'environ 400 000 g/mole.

**9.** Système thérapeutique transdermique selon l'une des revendications 7 à 8, **caractérisé en ce que** le premier polymère polybutène a un poids moléculaire moyen $M_n$ dans la plage de 700 à 2 800 g/mole et le deuxième polymère polybutène a un poids moléculaire moyen $M_n$ dans la plage de 2 200 à 6 500 g/mole.

**10.** Système thérapeutique transdermique selon l'une des revendications 1 à 9, dans lequel la couche de substance active contient 30 à 50% en poids de substance active et 50 à 70% en poids de matrice de polymère par rapport au poids total de la couche de substance active.

**11.** Système thérapeutique transdermique selon l'une des revendications 1 à 10, dans lequel la matrice polymère de la couche de substance active comprend au moins un polymère et/ou un copolymère sans groupes hydroxyle libres et sans groupes carboxyle libre, choisi dans le groupe comprenant des polyacrylates, des copolymères d'acrylate-acétate de vinyle, des polyisobutylènes, des copolymères de styrène-butadiène et des mélanges de ceux-ci.

**12.** Système thérapeutique transdermique selon l'une des revendications 1 à 11, **caractérisé en ce que** le STT présente sur une durée d'au moins 48 heures, un profil de perméation essentiellement linéaire de la substance active.

**13.** Procédé de production d'un système transdermique selon l'une des revendications 1 à 12, comprenant

i) la production d'un composant contenant la couche de substance active, contenant la couche de revêtement et la couche de substance active qui se trouve du côté de la couche de recouvrement qui est déterminée comme étant le côté tourné vers la peau ;
ii) la production d'un composant contenant la couche adhésive, contenant la couche pelliculable et la couche adhésive se trouvant sur la couche pelliculable et une membrane contrôlant la libération de la substance active ;
iii) la stratification des composants i) et ii) les uns sur les autres, de sorte que la couche de recouvrement et la couche pelliculable représentent en coupe du STT fini, les couches extérieures opposées.

**14.** Procédé selon la revendication 13, comprenant

i) l'application et éventuellement le séchage consécutif d'un film d'une composition formant la couche de substance active, éventuellement sous la forme d'une solution ou d'une dispersion dans un milieu approprié, sur le côté de la couche de recouvrement qui est déterminée comme étant le côté tourné vers la peau et éventuellement, le contrecollage avec la membrane contrôlant la libération de la substance active ;
ii) l'application et éventuellement, le séchage consécutif d'un film d'une composition formant la couche adhésive, éventuellement sous la forme d'une solution ou d'une dispersion dans un milieu approprié sur la couche de recouvrement et
iii) la stratification des composants des points i) et ii) les uns sur les autres, de sorte que la couche de recouvrement et la couche pelliculable représentent en coupe du STT fini, les couches externes opposées;

ou

i) l'application et éventuellement le séchage consécutif d'un film d'une composition formant la couche de substance active, éventuellement sous la forme d'une solution ou d'une dispersion dans un milieu approprié, sur un film pelliculable (« revêtement intermédiaire » siliconé ou revêtu de fluoropolymère et le contrecollage avec la couche de recouvrement ;
ii) le retrait du film pelliculable siliconé (« revêtement intermédiaire » et le contre-collage de la membrane contrôlant la libération de la substance active ;
iii) l'application et éventuellement, le séchage consécutif d'un film d'une composition formant la couche adhésive, éventuellement sous la forme d'une solution ou d'une dispersion dans un milieu approprié sur la couche pelliculable et
iv) la stratification des composants des points ii) et iii) les uns sur les autres, de sorte que la couche de recouvrement et la couche pelliculable représentent en coupe du STT fini, les couches externes opposées.

**15.** Système thérapeutique transdermique selon l'une des revendications 1 à 14, pour son utilisation dans le traitement de la démence de type Alzheimer et la démence parkinsonienne.

Figur 1

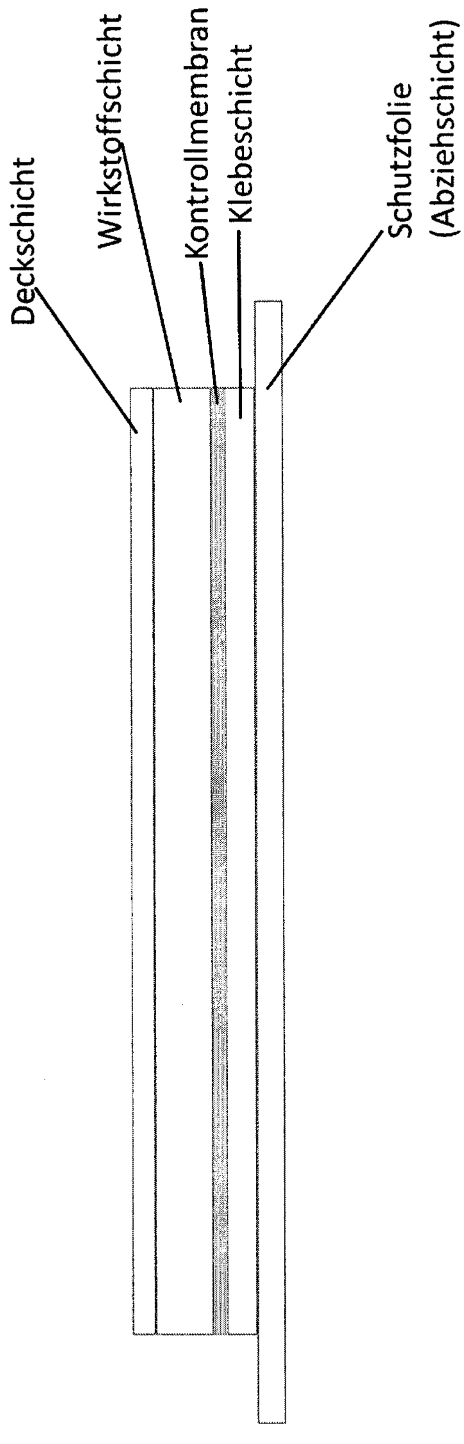
Deckschicht
Wirkstoffschicht
Kontrollmembran
Klebeschicht
Schutzfolie
(Abziehschicht)

Figur 2

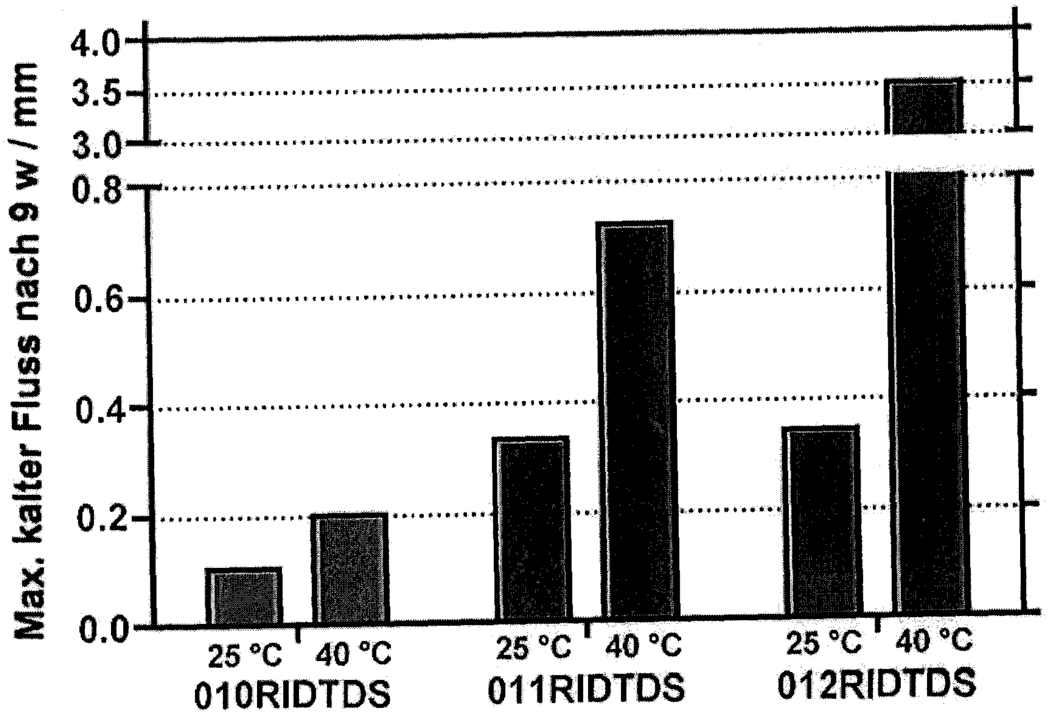
Max. kalter Fluss nach 9 w / mm
4.0
3.5
3.0
0.8
0.6
0.4
0.2
0.0
25 °C
40 °C
010RIDTDS
25 °C
40 °C
011RIDTDS
25 °C
40 °C
012RIDTDS

Figur 3

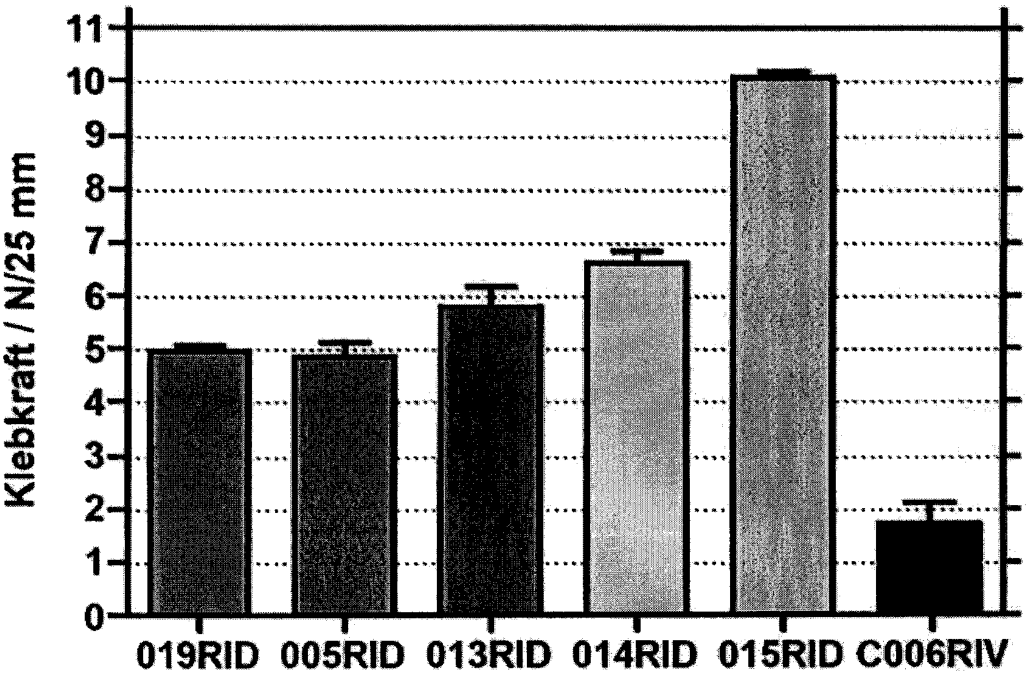
Klebkraft / N/25 mm
11
10
9
8
7
6
5
4
3
2
1
0
019RID
005RID
013RID
014RID
015RID
C006RIV

Figur 4

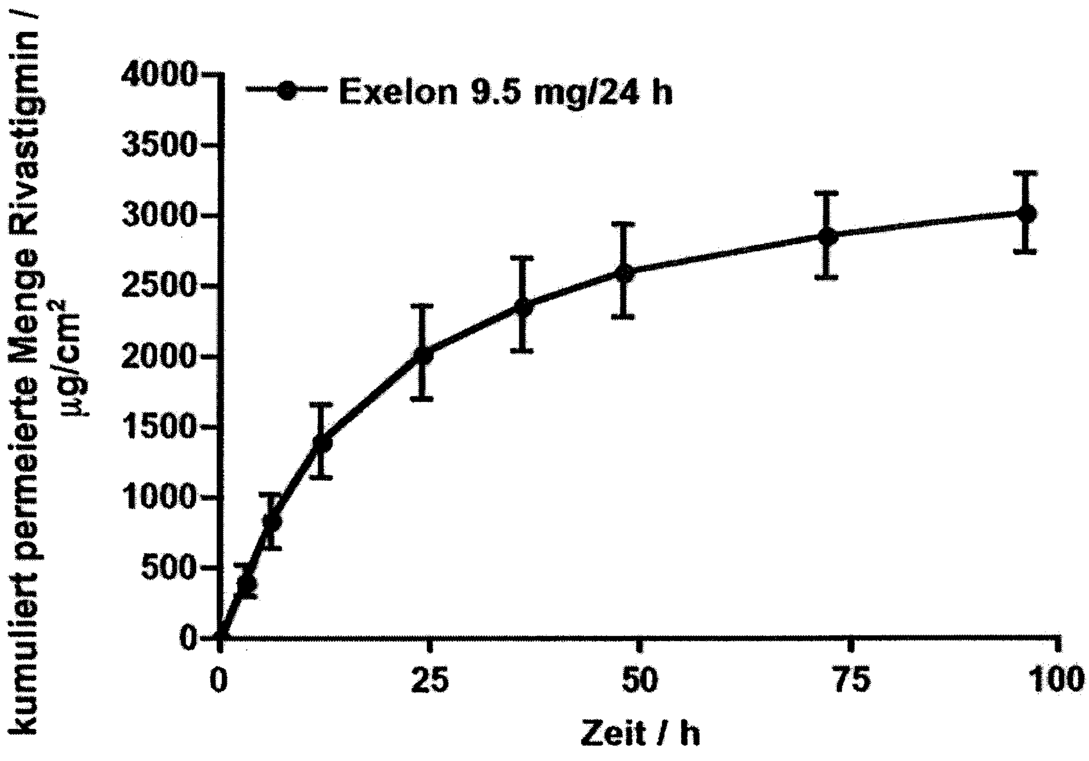
kumuliert permeierte Menge Rivastigmin / µg/cm²
4000
3500
3000
2500
2000
1500
1000
500
0
0
25
50
75
100
Zeit / h
Exelon 9.5 mg/24 h

Figur 5

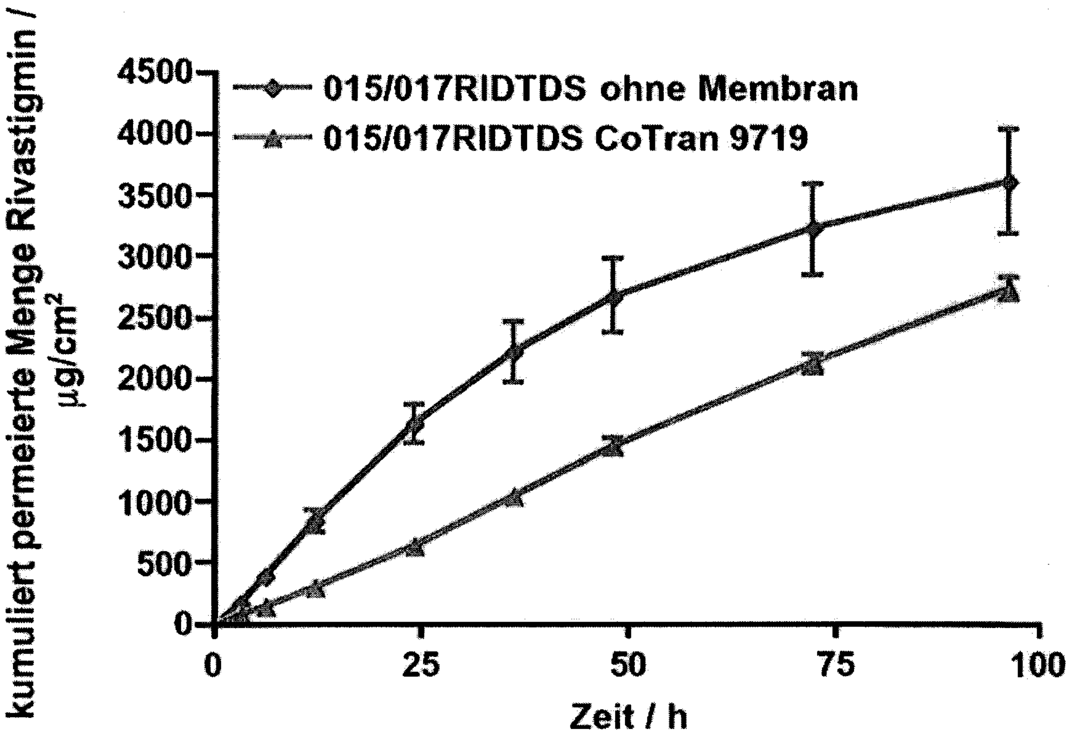
kumuliert permeierte Menge Rivastigmin / µg/cm²
4500
4000
3500
3000
2500
2000
1500
1000
500
0
0
25
50
75
100
Zeit / h
015/017RIDTDS ohne Membran
015/017RIDTDS CoTran 9719

Figur 6

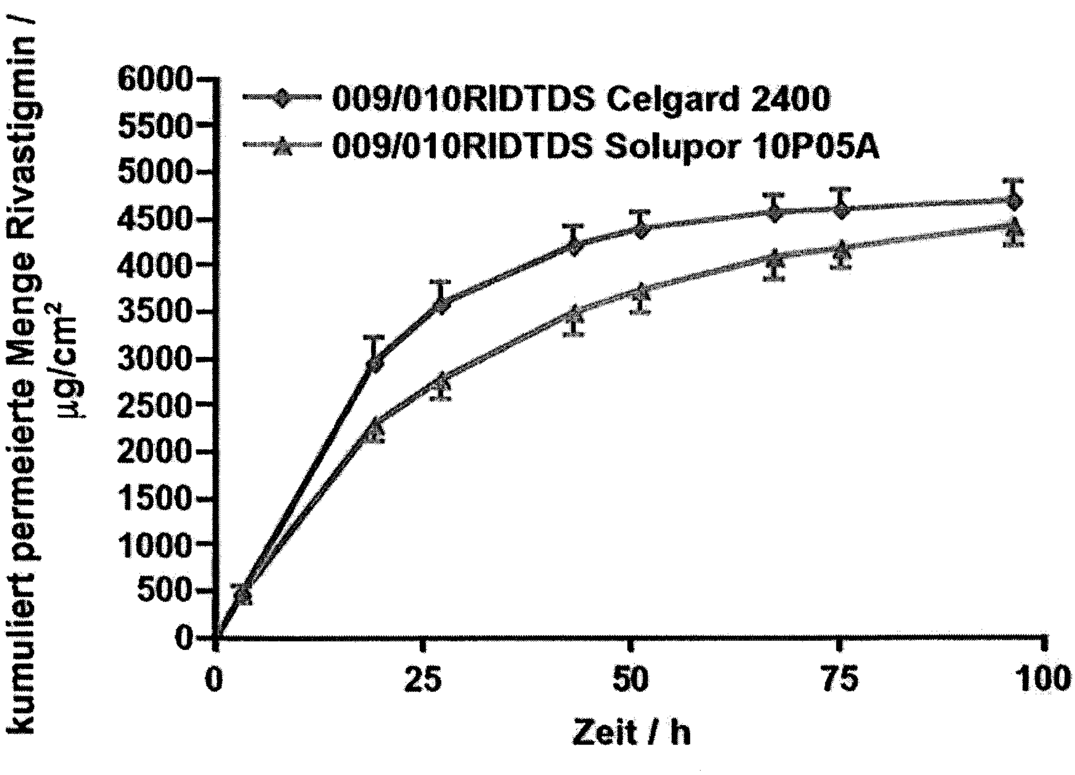
kumuliert permeierte Menge Rivastigmin / µg/cm²
6000
5500
5000
4500
4000
3500
3000
2500
2000
1500
1000
500
0
0
25
50
75
100
Zeit / h
009/010RIDTDS Celgard 2400
009/010RIDTDS Solupor 10P05A

Figur 7

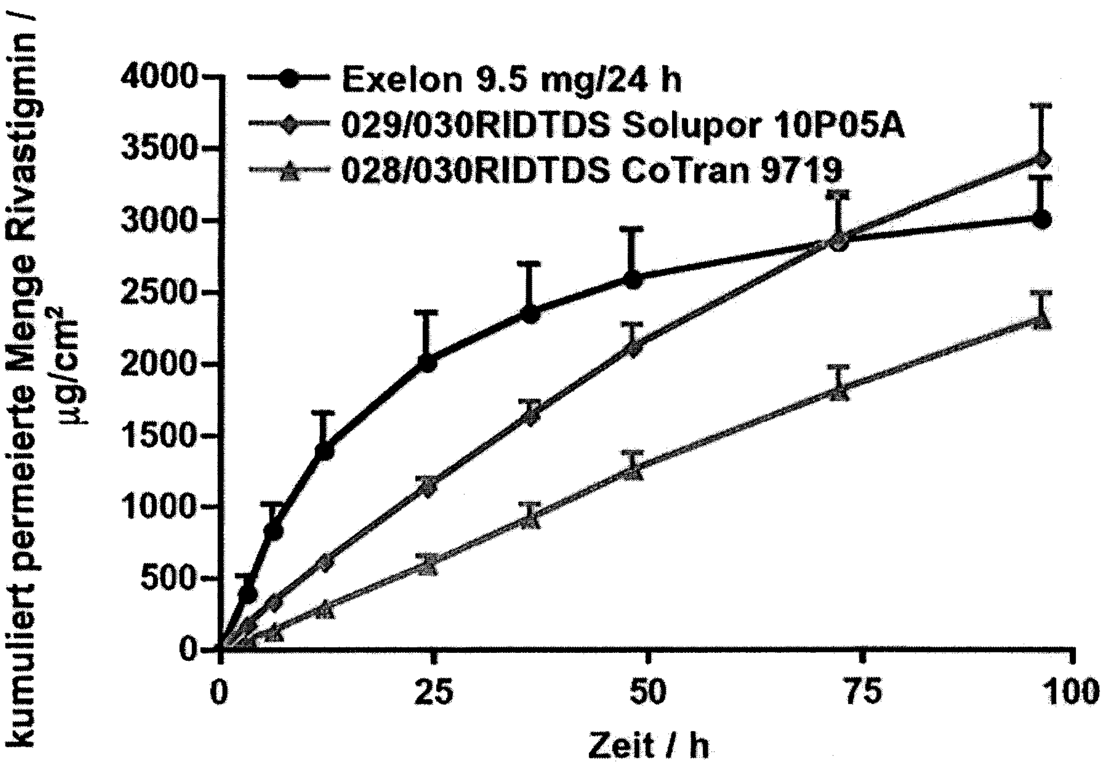
kumuliert permeierte Menge Rivastigmin / µg/cm²
4000
3500
3000
2500
2000
1500
1000
500
0
0
25
50
75
100
Zeit / h
Exelon 9.5 mg/24 h
029/030RIDTDS Solupor 10P05A
028/030RIDTDS CoTran 9719

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- GB 2203040 A
- WO 0203969 A
- WO 2008021113 A
- EP 2016939 A
- DE 19918106
- WO 2007064407 A1
- US 20080044461 A1
- US 20070259028 A1
- US 20040086552 A1
- US 6689379 B1
- US 20080175890 A1
- WO 9404109 A1
- EP 1047409 A
- WO 03017988 A1
- WO 03017988 A